(19) Europäisches Patentamt
European Patent Office
Office européen des brevets

(11) Publication number: **0 175 577 A2**

(12) **EUROPEAN PATENT APPLICATION**

(21) Application number: **85306646.2**

(22) Date of filing: **18.09.85**

(51) Int. Cl.[4]: **G 01 N 33/58**, G 01 N 33/542, G 01 N 33/543, G 01 N 33/72, C 12 Q 1/32 // G01N33/569, G01N33/576

(30) Priority: **21.09.84 US 652856**

(43) Date of publication of application: **26.03.86 Bulletin 86/13**

(84) Designated Contracting States: **AT BE CH DE FR GB IT LI LU NL SE**

(71) Applicant: **Kiel, Jonathan L, 5814 Fort Stanwix, San Antonio Texas 78233 (US)**

(72) Inventor: **Kiel, Jonathan L, 5814 Fort Stanwix, San Antonio Texas 78233 (US)**

(74) Representative: **McCallum, William Potter et al, Cruikshank & Fairweather 19 Royal Exchange Square, Glasgow G1 3AE Scotland (GB)**

(54) **Microchemiluminescent assay system.**

(57) A composition for use in a thermochemiluminescent assay. The composition includes a heme protein, a luminescer such as luminol, and serum albumin, the luminescer being noncovalently bonded to serum albumin. The composition may be used in a thermochemiluminescent for the ligant of a specific binding pair in which the composition is contacted with a fluid sample suspected of containing the antiligand of the specific binding pair and the subsequent light-emitting reaction is quantified. The specific binding light-emitting reaction is quantified. The specific binding pair can be, for instance, an enzyme and the substrate for which it is specific. The composition and the assay can be modified to detect ionizing radiation, peroxidative injury to cells, heat or a particular cell type.

EP 0 175 577 A2

# MICROCHEMILUMINESCENT ASSAY SYSTEM

The present invention relates to the use of luminescent reactions to detect the presence and/or quantity of heat, ionizing radiation or specific substances such as a cell type, antigen/antibody, or an enzyme or its substrate. In more detail, the present invention relates to methods for the use of a complex formed from serum albumin and a luminescer which, in the presence of heat, ionizing radiation, or a specific analyte, will emit light in an amount proportional to the amount of the heat, ionizing radiation or analyte present.

The present invention is based on the co-pending application "Insoluble Crosslinked Cytotoxic Oxidase-Peroxidase System", hereinafter designated by the abbreviation "ICCOPS", Serial No. 251,694, filed on April 7, 1981. That invention is directed to a composition comprising two cross-linked enzymes, one an oxidase, the other a peroxidase, and the use of that composition for the control of tumor growth and immune diseases. A preferred embodiment of that invention is to cross-link the peroxidase with the oxidase in glutaraldehyde and to use bovine serum albumin as the insoluble support for the cross-linked enzymes. This composition appeared to function by oxidizing the cellular components of the target cells.

A number of reactions involving peroxidases in luminescent oxidations are known in the art (see, for instance U.S. Patent Nos. 3,099,605, 3,564,588, 3,880,934, 4,353,984 and 4,391,904). These known luminescent oxidation reactions prompted Applicant to use the ICCOPS system in a luminescent reaction in which the luminescer was oxidized rather than the cellular components which are oxidized using the ICCOPS according to the teachings of that co-pending application. This use of the ICCOPS in a luminescent reaction requires that the oxidase catalyze the production of hydrogen peroxide which, in turn, oxidizes the luminescer. The oxidation of the luminescer is catalyzed by peroxidase.

The microchemiluminescent assay system (hereinafter designated by the abbreviation "MCLAS") of the present invention is based on the discovery that luminol, luminol derivatives, and other aromatic or hydrophobic luminescent compounds will bind in a non-covalent bond to serum albumin, eliminating the catalyst, such as peroxidase, and oxidase required by the ICCOPS while retaining their luminescent properties with a high efficiency. In fact, not only will these compounds retain their luminescent properties, but serum albumin actually appears to function as an oxidase and a peroxidase as well as a catalyst which increases the efficiency of the luminescent reaction.

The combination of luminol, luminol derivatives, or aromatic or hydrophobic luminescent compounds with a protein is not new. For instance, United States Patent No. 4,104,029 discloses the covalent bonding of luminol and other luminol derivatives to

various proteins through the amino group of the luminol or luminol derivative. In particular, that patent discloses the covalent bonding of luminol to insulin through the amino group of luminol using glutaraldehyde as a coupling agent. However, glutaraldehyde will not promote the linking of serum albumin to luminol, or luminol derivatives, and no indication is given by that patent as to a method appropriate for doing so.

British Patent No. 2,008,247 discloses the covalent bonding of luminol to an antibody through a peptide bond and the light emitting reaction which occurs in the presence of red blood cells. The hemoglobin of the red blood cell catalyzes the formation of hydrogen peroxide which, in turn, oxidizes the luminol. This reference also suggests that the chemiluminescent reaction can be triggered by a change in pH, temperature, solvent polarity or by incident ionizing radiation, but provides no indication of how these results may be accomplished or the possible utility of such a system.

Further, there appears to have been no reason to attempt to form a complex between serum albumin and luminol or luminol derivatives because it has been known in the art that serum albumin acts as an inhibitor to luminescent reactions. For instance, Schroeder, H.R., et al. ("Monitoring Specific Protein-Binding Reactions with Chemiluminescence," 57 Methods in Enzymology 424, at 424 and 439 (1978)), and the references cited therein, note that bonding of an alkyl group to the amino residue of luminol causes a substantial decrease in the efficiency of

luminol as a light emitter. Others have noted that covalent bonding to the amino groups on luminol and isoluminol may reduce luminescence by as much as a factor of 100. Zellner, C.N., et al., 59 J. American Chemical Society 2580 (1937) and Branchini, B.R., et al., 111 Analytical Biochemistry 87 (1981). Consequently, it would be expected that, even if serum albumin were linked to luminol, it would quench the luminescent reaction.

There have been reports of the catalytic action of serum albumin in the past. For instance, it has been reported that bovine serum albumin possesses the ability to catalyze the cis-trans reaction of photochromic azobenzene compounds. Lovrien R., et al., 6 Biochemistry 2281 (1967). In a system unrelated to luminescent reactions, it has been reported that bovine serum albumin is a good catalyst for driving the decomposition of Meisenheimer complexes, but that human serum albumin is inert. Taylor, R., et al., 95 J. Am. Chem. Soc. 5819 (1973); see also Sturgill, et al., 485 Biochim. Biophys. Acta 236 (1977). It has also been reported that the addition of crystalline bovine serum albumin to a solution containing luminol resulted in a fifteen-fold increase in fluorescence polarization of luminol. Prichard, P.M., et al., 31 Biochem. Biophys. Res. Comm. 131 (1968).

The prior art also discloses various light-emitting reactions as part of an assay for a specific analyte. Often these methods rely on the presence of $H_2O_2$ in order to oxidize the luminescer, therefore they couple a reaction catalyzed by an oxidase with a peroxidase-catalyzed oxidation/reduction reaction. These

reactions have been described by many in the literature, a summary appearing at Seitz, W.R., Chemiluminescence Detection of Enzymically Generated Peroxide, 57 Methods in Enzymology 445 (1978).

The discovery that serum albumin acts as a catalyst in light generating reactions when non-covalently bonded to a luminescer creates many possibilities for the use of this complex in a luminescent assay for the detection of heat, ionizing radiation and many specific analytes. All that is needed is a means for triggering the light-emitting reaction. In the case of the serum albumin-luminol complex of the present invention, all that is needed to trigger the luminescent reaction of the serum albumin-luminol or a serum albumin-luminol derivative complex is the addition of a base. If the serum albumin-luminescer complex is coupled to a heme protein, all that is needed to trigger the light-emitting reaction is the addition of heat. If the heme protein-serum albumin-luminescer conjugate is coupled to an oxidizing enzyme, all that is needed to trigger the luminescent reaction is the presence of the substrate specific for that oxidizing enzyme.

The simplicity and ready availability of the various components of these systems imparts to the present invention a number of characteristics which represent advantages over previous assay methods. For instance, for many years it has been a practice to test for the presence of glucose in certain body fluids by the chemiluminescent method disclosed by U.S. Patent No. 3,099,605,

issued to Free, or by the improvements which have been made to that technique as disclosed by the literature and other patents subsequent to that patent. The relatively low sensitivity of this method, and the fact that it is difficult to quantify the amount of glucose present using this method are limitations which affect the utility of the method. These limitations can be overcome using the present invention.

Another commonly used analytical method is the method which is often referred to as a radioimmunoassay or RIA. The RIA is characterized by the fact that it uses radioactive chemicals. The radioactivity of these chemicals creates problems in the handling, storage and disposal of these reagents. The present invention offers the specificity of an RIA without the attendant problems such as the cost and limited availability of the reagents, the problems in storage, handling and disposal of the reagents and the necessary steps of separation and transfer which characterize the RIA.

Like the RIA, bioluminescent assays are very sensitive, and have the advantages of not requiring a high level of skill on the part of the operator and not creating handling, storage and disposal problems. However, very little work has been done with them such that there are no standards and comparisons from one method to the next have not been made.

The enzyme-linked immunosorbent assay (ELISA) is highly sensitive, relatively quick to run, can be automated so that many samples can be assayed simultaneously, and uses relatively small

volumes of reagents. However, the ELISA is characterized by difficulties in reproducing results from like samples and the requirement for a high skill level on the part of the operator. Further, there has been little attempt to standardize enzyme-antibody conjugates or to determine the efficiency of the coupling reaction. Fluorescent antibody assays are also very sensitive and require only small quantities of reagents. However, they also require a high skill level on the part of the operator, have many controls and are time consuming. Further, they are semi-quantitative at best, and sometimes only qualitative in nature.

It has also been known to use a luminol-labeled analyte in a chemiluminescent assay for an antibody. For instance, Hersh, C.S., et al., "A luminol-assisted competitive binding immunoassay of human immunoglobulin G" (in Methods in Enzymology, S.P. Colowick and N.O. Kaplan, Eds., Vol. 73, Immunochemical Techniques (1981), pp. 608-615), describes a precipitin reaction in which the precipitate is tested for chemiluminescence, and the modification of the method for use in nonprecipitating antibody-antigen complexes by the use of either a second antibody or a separating agent. In more detail, this method involves the covalent bonding of luminol to IgG and the mixing of the luminol-IgG conjugate with an anti-IgG antigen to form a precipitate which is then contacted with hemin and $H_2O_2$ to generate light.

The method described by Hersh, et al., supra, has been applied to the coupling of luminol to other immunoglobulins, to human chorionic gonadotropin (HCG) and to the coupling of diazotized luminol to bovine serum albumin and HCG, the last coupling being accomplished by a modification of the method described by Simpson, J.S.A., et al., 279 Nature (London) 646 (1979). The luminol-HCG conjugate formed according to that modified method contained approximately ten luminol molecules covalently linked to each HCG molecule. The chemiluminescent efficiency of these conjugates was decreased compared to the efficiency of the luminol-IgG conjugate, and in the case of the luminol-diazo-HCG conjugate, the efficiency of the light emitting reaction was 0.5-4% of the efficiency of the reaction involving luminol-IgG.

The sensitivity, flexibility, inexpensive nature of the reagents and low skill level required of the operator confer upon the present invention advantages which are not present in any of these prior art assay methods. For instance, serum albumin is relatively inexpensive, is readily available, and is relatively inert to many biochemical compounds. An example of its sensitivity is the fact that an assay conducted according to the present invention involving a lectin-serum albumin-luminol complex on red blood cells has a sensitivity limit of approximately 80 attomoles (0.08 femtomoles) of reagent when activated by a base such as sodium hydroxide. The best sensitivity achieved with conventional assays such as the $H_2O_2$-microperoxidase or the

$H_2O_2$-hematin peroxidase system is 150 attomoles (0.15 femtomoles) at a picomolar concentration. Luminol is also readily available and inexpensive. Neither serum albumin nor luminol present significant problems in handling or storage. The present invention is highly flexible, being adaptable for use in the detection of a wide variety of biologicals, including many which are not antigenic. Further, it can be adapted to provide, for the first time, a rapid assay for cell-mediated immunity. Current methods of testing for hypersensitivity involve measuring the incorporation of tritiated thymidine into the DNA of lymphocytes which have been previously stimulated by an antigen or mitogen. The tritiated thymidine assay requires three days of incubation and the use of radioactive materials. Using the method of the present invention, which requires no radioactive material, the assay can be run in an hour. Results obtained using the present invention are consistently reproducible and can be attained quickly and without exhaustive training of the operator.

## Summary of the Invention

The present invention is directed to a method for detecting ionizing radiation, heat and peroxidative injury to cells with the serum albumin-luminescer complex.

The present invention also describes a conjugate for use in a chemiluminescent assay comprising a heme protein, serum albumin and a luminescer, the heme protein being bonded to the serum albumin and the luminescer being non-covalently bonded to the

serum albumin. The heme protein-serum albumin-luminescer conjugate can also be used as a composition and in a method for detecting heat. A device may be made incorporating the heme protein-serum albumin-luminescer conjugate for the detection of heat or ionizing radiation.

The present invention is also directed to a composition and method for the detection and quantification of a ligand of a specific binding pair wherein said specific binding pair consists of a ligand and an antiligand comprising a complex formed from the bonding of a ligand to the serum albumin, the bonding of a heme protein to said serum albumin, and the non-covalent bonding of a luminescer to the serum albumin and the use of that complex to bind to the antiligand of the specific binding pair.

The present invention also describes a composition and method for detecting possible injury to cells caused by stimuli which can cause peroxidation of cellular components.

It is, therefore, an object of the present invention to provide a method and composition for the detection and quantification of heat or ionizing radiation.

Another object of the present invention is to provide a method and composition for the detection and quantification of peroxidative injury to cells.

Another object of the present invention is to provide a device for the detection and quantification of heat or ionizing radiation.

Another object of the present invention is to provide a method and a composition for use in that method for the detection and quantification of a specific analyte where that specific analyte is a member of a specific binding pair.

Another object of the present invention is to provide a device for the detection and quantification of a specific analyte where that specific analyte is a member of a specific binding pair.

Another object of the present invention is to provide a method and a composition for use in that method for the detection and quantification of a specific cell type.

Another object of the present invention is to provide a device for the detection and quantification of a specific cell type.

Another object of the present invention is to provide a chemiluminescent assay capable of being used to detect and quantify a specific cell type in a fluid or semi-solid containing multiple cell types or to detect and quantify a specific analyte in a fluid or semi-solid where that specific analyte is a member of a specific binding pair.

Still another object of the present invention is to provide a reliable, highly sensitive, simple method, and a composition for use in that method, for the detection of a specific ligand where that ligand is a member of a specific binding pair which is inexpensive and easily adaptable for use in the detection of various ligands.

Another object of the present invention is to provide a composition for use in a chemiluminescent immunoassay which is inexpensive to prepare and which can be used with a variety of antibody/antigen pairs.

These and other objects of the present invention, which will become apparent throughout this disclosure, are accomplished according to the following detailed description.

Brief Description Of The Drawings

Fig. 1 is a graph showing the thermochemiluminescence of cross-linked hemoglobin-bovine serum albumin (BSA) luminol gel. The results are from 1 milliliter (ml) of gel suspension heated in a water bath and immediately counted in a Beckman LS230 scintillation counter in the out-of-coincidence mode.

Fig. 2 is a graph showing the thermochemiluminescence of horseradish peroxidase-bovine serum albumin-luminol labeled erythrocytes of various species. Closed circles represent Landrace-Duroc crossbred porcine cells, the open circles represent miniature porcine cells, open squares represent Sprague-Dawley rat cells, and the closed squares represent human red blood cells. The solid line is the linear regression for the Landrace-Duroc data, the line of alternating dots and dashes is the linear regression line for the rat data, and the dashed line is the linear regression line for the pooled miniature swine and human data.

Fig. 3 is a graph showing the thermochemiluminescence of red blood cells (RBCs) from Poland-China pigs. The open circles

represent RBCs labeled with luminol, bovine serum albumin (BSA) and horseradish peroxidase (HRP), the closed circles represent RBCs labeled with BSA and HRP, and the open squares represent RBCs tanned with glutaraldehyde.

Fig. 4 is a graph showing the thermochemiluminescence of luminol-bovine serum albumin-horseradish peroxidase labeled human red blood cells (RBCs). The closed squares represent data of normal human RBCs, open circles represent data of normal RBCs exposed to halothane, the closed circles represent RBCs treated with 1-chloro-2,4-dinitrobenzene (CDNB), and the open squares represent CDNB-treated RBCs exposed to halothane.

Fig. 5 is a graph showing the effect of halothane exposure on the thermochemiluminescence of red blood cells from Landrace-Duroc crossbred pigs labeled with luminol and horseradish peroxidase. The open circles represent data from untreated porcine red blood cells, and the closed squares represent data from porcine red blood cells exposed to halothane.

Fig. 6 is a graph showing the chemiluminescence of fractions from a G-25 Sephadex column (48 ml) loaded with a 2 ml solution of crosslinked concanavalin A (Con A) and bovine serum albumin (BSA) containing absorbed luminol. Crosslinking was stopped by addition of 100 mg of glycine.

Fig. 7 is a graph showing the chemiluminescence of murine splenocytes from mice which were previously sensitized with antigen. The closed squares represent data from the control group, open circles represent data from cells which were tested two days

after sensitization and the open squares represent data from cells tested four days after sensitization.

Detailed Description of the Invention

The present invention relates to the chemiluminescence of certain compounds, designated generically as luminescers, upon oxidation. One of the most common luminescers is luminol (5-amino-2,3-dihydrophthalazine-1,4-dione), which will luminesce upon the addition of a strong base, an oxidizing agent and a catalyst. There are many derivatives of luminol which also act as luminescers, including isoluminol and diethylisoluminol, which are two of the more common of the luminol derivatives. The term "luminol derivatives" refers to luminol and those compounds formed by the substitution of various substituent (R) groups onto the aminophthalhydrazide backbone ring of luminol. Those luminol derivatives which are most active as luminescers are those in which the substituent group is attached through an alkyl bridging group to the amino residue of isoluminol. These derivatives include but are not limited to, the following:

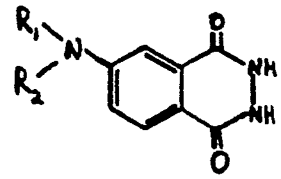

Aminophthalhydrazide

| | $R_1$ | $R_2$ |
|---|---|---|
| Isoluminol | -H | -H |
| Diethyl-isoluminol | $-CH_2CH_3$ | $-CH_2CH_3$ |
| (I) | -H | $-CH_2CH(OH)CH_2NH_2$ |
| (II) | -H | 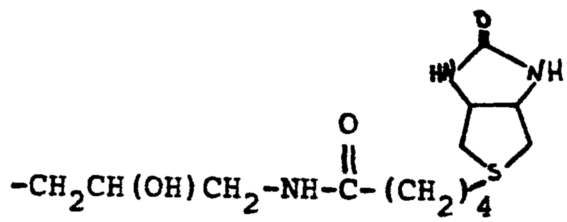 |
| (III) | $-CH_2CH_3$ | $-CH_2CH(OH)CH_2NH_2$ |
| (IV) | $-CH_2CH_3$ | 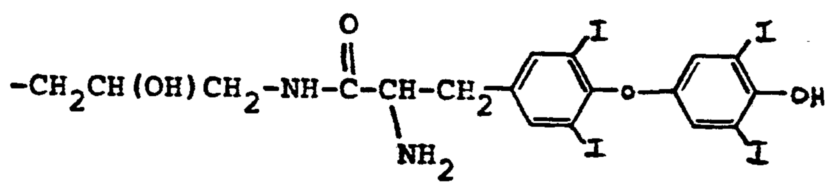 |
| (V) | -H | $-(CH_2)_4NH_2$ |
| (VI) | $-CH_2CH_3$ | $-(CH_2)_4NH_2$ |
| (VII) | $-CH_2CH_3$ | 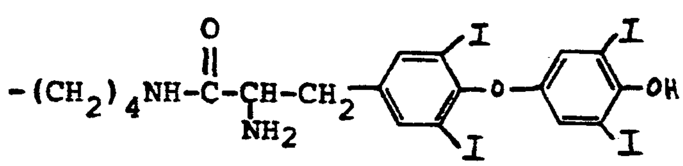 |
| (VIII) | 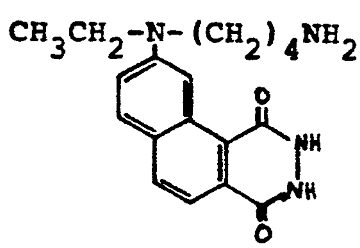 | |

(IX) 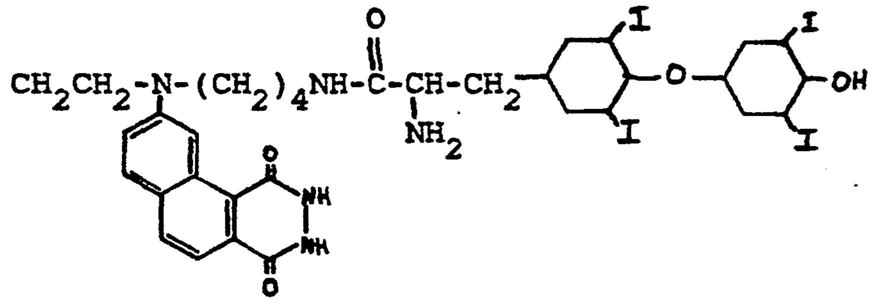

Methods for the preparation of these luminol derivatives are summarized in Schroeder, H.R., et al., 57 Methods in Enzymology 425-445 (1978), hereby incorporated by reference. Other compounds which act as luminescers when used in the present invention include, but are not limited to, phenol pyruvate, indole-3-pyruvate, tryptophan, N-acetyltryptophan or other tryptophan derivatives, indole propionic acid, and indole acetic acid. The light emitted upon oxidation of the luminescer can be measured in a luminometer or scintillation counter, such as the Model 3000 integrating luminometer made by Science Applications, Inc. or the Beta liquid scintillation counter, Model LS230, made by Beckman Instruments.

The various derivatives of luminol show considerable differences in their chemiluminescent activity. For instance, aminonaphthylhydrazides and the thyroxine-naphthylhydrazide conjugate (IX in the above table) can be detected at lower levels than the aminophthalhydrazide derivatives of luminol. Schroeder, et al., <u>supra</u> at p. 440. The non-luminol derivatives capable of

acting as luminescers listed above also show some variation in their luminescent properties.

Further, the light emitting reaction is dependent upon pH. Luminol in particular is very sensitive to changes in pH, being capable of its most efficient chemiluminescence at a pH of approximately 10 and being only sparingly soluble in water at a pH of less than 8. The pH dependency of the light emitting reaction and the solubility characteristics of luminol are factors which have limited the usefulness of chemiluminescent techniques as assays in the past.

Serum albumin is the most abundant of the plasma proteins, and one of its functions is to act as a transport for many sparingly soluble metabolic products. The tertiary structure of serum albumin is such that a pocket is created on one side of the molecule. It is this pocket which helps albumin serve its transport function because the pocket possesses great affinity for aromatic and/or hydrophobic molecules. Luminol, and many of its derivatives, are aromatic and/or hydrophobic, as are many of the other molecules capable of acting as luminescers listed above. The present invention is based upon the discovery that luminol, luminol derivatives, and non-luminol based luminescers will non-covalently bond with serum albumin in this pocket, and that such non-covalent bonding, rather than quenching the luminescent reaction, will result in the albumin acting as a very efficient catalyst of the luminescent reaction. Further, it is such an efficient catalyst that the above-listed compounds other

than luminol may also be used as luminescers when they are non-covalently bound to serum albumin in this manner.

The term "serum albumin", as used herein, refers to a protein of MW = 68,000 - 69,000 which is the major protein component of blood plasma. Although the methods and compositions of the present invention all refer to bovine serum albumin (BSA), it will be understood by those of skill in the art who have the benefit of this disclosure that human or serum albumin from other sources may also be used to advantage. In fact, there is some evidence indicating that the ability of serum albumin to function as a catalyst, such as it apparently does in the present invention, may vary according to its source. For instance, Taylor, et al., 6 Biochemistry 2281 (1967) reported that bovine serum albumin exhibited catalytic activity in certain applications while human serum albumin was inert.

The term "bifunctional cross-linking agent", as used herein, refers to an agent which is capable of effecting the polymerization of the protein components of the assay system of the present invention. For instance, the methods set forth in the examples below all disclose the use of either glutaraldehyde or N-succinimidyl-3(2-pyridyldithio)-propionate as cross-linking agents to gel the reagents. Other bifunctional cross-linking agents which may be similarly utilized include the carbodiimides, such as 1-ethyl-3-(3-dimethyl-aminopropyl) carbodiimide, succinyl anhydride, and those described by Brown, G.B., 44 Methods in Enzymology 263-280 (1970), hereby incorporated by reference.

The term "heme protein", as used herein, refers to a biomolecule which is capable of participating in a reaction to catalyze the production and utilization of oxidizing agents such as superoxide and peroxide in the oxidation of a luminescer. Heme proteins which may be used to advantage in accordance with the present invention include, but are not limited to, hemoglobin, myoglobin, catalase and peroxidase. The peroxidase can be horseradish peroxidase, lactoperoxidase, microperoxidase, or any other peroxidase. The term "heme protein" also refers to materials such as red blood cells, which contain hemoglobin, and which can function as a heme protein when cross-linked in a gel in accordance with the teachings of the present invention.

The term "polyamino compound", as used herein, refers to a biomolecule which has two or more amino groups available such that it is capable of conjugation with the aldehydic moiety of a specific ligand and with the albumin of the serum albumin-luminescer complex. Polyamino compounds which may be used to advantage in accordance with the present invention include, but are not limited to, lysine, hexene diamine, spermidine, ethylene diamine and putresine (1,4 - butane diamine).

The luminescent reactions of the present invention can also be adapted for use in energy transfer gel (ETG) systems. ETG systems are systems composed of semi-synthetic or synthetic enzymes in a gel matrix which are capable of converting electromagnetic energy (ranging from heat to ultraviolet to

ionizing radiation) into chemical energy or into another energy level of electromagnetic radiation (i.e., a change in wavelength). An ETG is composed of a polymeric form of enzyme, a matrix protein or synthetic form of enzyme, a matrix protein or synthetic polymer, an entrapped substrate, and an entrapped (bound) solvent. In some simplified forms, the polymeric matrix also would act as the catalyst.

The gel state is maintained by interactions between the solvent and polymeric matrix. For instance, the matrix may be negatively charged and the solvent either aprotic (such as dimethylsulfoxide or acetone) or protic (water) or a combination of the two. Fully swollen negative gels may be maintained by hydrogen ion pressure (T. Tanaka, Gels, Scientific American, Jan. 1981, pp. 124-138). This pressure is the force created by the bombardment of the negatively charged matrix polymer molecules by the hydrogen ions at a given temperature. The hydrophobic bonding of the polymeric matrix and the hydrogen ion pressure are two opposing forces which maintain the state of the gel. The gel can be collapsed by lowering the temperature of the gel or by decreasing the hydrogen ion content by electrochemically neutralizing the negatively charged matrix.

If a high concentration of hydrogen ions is present in the matrix, lowering the temperature or subjecting the gel to a static electric field will collapse the gel in a continuously exponential manner. If the hydrogen ion concentration is decreased to a critical level by substitution of some of the

protic solvent with aprotic solvent, then an infinitesimally small change in either the input or output of energy will cause an abrupt change in the gel volume. These conditions generate a true phrase transition. T. Tanaka, I. Nishio, S-T Sun, and S. Ueno-Nishio, Collapse of Gels in an Electric Field, 218 Science 467-469 (1982). This change is evidenced physically by large changes in volume that occur over several hours.

The negatively charged matrix may also be neutralized and crosslinked and hydrogen ions may be excluded from the matrix by divalent cations such as calcium or magnesium ions. The result is the same: the gel collapses.

If the gel is brought to the brink of a transition, either in physical phase or chemical activity, that is prevented by a thermodynamic barrier (transition enthalpy), then the input or output of this energy will result in an abrupt change. If a chemical reaction is to be initiated or accelerated in this manner, it should be kinetically favored.

A complex formed from luminol and bovine serum albumin (BSA) forms a basic building block which may be modified for use in an ETG system according to the teaching of the present invention. For instance, the BSA-luminescer complex can be used in an ETG system as an assay for detection of malignant hyperthermia. This assay is made possible by the fact that oxyhemoglobin undergoes spontaneous autoxidation in red blood cells (RBCs), generating superoxide and subsequently, hydrogen peroxide and lipid peroxides. The autoxidation is minimized in the RBC by various

endogenous enzymes such as superoxide dismutase, catalase, and glutathione peroxidase, which catalyze the breakdown of these oxides. A deficiency in these enzymes can be detected by preparing a gel by mixing RBCs from the patient with a bifunctional cross-linking agent, then adding serum albumin, a heme protein and a luminescer. The gel is theh heated, and a roughly exponential increase in the thermochemiluminescence of the heated gel with increased temperature is a positive indication that the patient suffers from one or more of the deficiencies associated with malignant hyperthermia. The thermodynamic barrier for activation of the chemiluminescent reaction may be lowered by the addition of a base to the gel.

This method may be simplified by the conjugation of the BSA-luminescer complex to a lectin. Lectins are proteins or glycoproteins which have a particular affinity for certain carbohydrates found on the surface of certain cell types, in particular, RBCs, white blood cells and tumor cells. Many are from plants, but some are isolated from animals such as the vineyard snail (Hetrix pomatia). Conjugating the lectin to the serum albumin-luminescer complex facilitates cross-linking in the ETG by increasing the affinity of the serum albumin-luminescer complex for the red blood cells. Increased thermochemiluminescence of this preparation at higher temperatures is an indicator of malignant hyperthermia.

Conconavalin A is a lectin which has particular affinity for RBCs and T cell lymphocytes and which has been used to advantage

in the present invention. Other lectins which may be used, depending on the cell type being used in the assay, include, but are not limited to, soybean lectin, wheat germ lectin (agglutinin), Helix pomatia lectin and lentil lectin.

By using different lectins in combination with the serum albumin-luminescer complex, an assay may be designed which is capable of use in assays other than for the detection of malignant hyperthermia. For instance, a thermochemiluminescent assay can be designed to measure thermosensitivity, genetic defects, nutritional deficiencies or exposure to toxic compounds or autoxidizable drugs, all of which may manifest themselves as deficiencies in the activities of endogenous catalase, superoxide dismutase, glucose-6-phosphate dehydrogenase, glutathione and glutathione reductase, or as deficiencies in vitamin E, vitamin A or vitamin C. Such an assay is conducted by coating RBCs with a lectin-serum albumin-luminescer conjugate, and heating the coated RBCs. Excess thermochemiluminescence is a positive indicator of such a deficiency or damage resulting from the exposure to an autoxidizable chemical. Such an assay has particular utility as a test for sensitivity to autoxidizable drugs by conducting the assay in the presence of such a drug. Among the drugs for which such sensitivity testing can be conducted are:

<u>Toxins</u>
Paraquat

<u>Anesthetics</u>
Halothane

Analgesics

Acetanilid
Acetophenetidin (phenacetin)
Acetylsalicylic acid
Acetaminophen

Anticonvulsants

Phenytoin
Phenacemide
Phenobarbitol
Carbamazepine
Mephenytoin

Anorectics

Chlorphentermine

Sulfonamides and Sulfones

Sulfanilamide
Sulfapyridine
Diaphenylsulfone
Thiazolsulfone
N-acetylsulfanilamide
Salicylazosulfapyridine (Azulfadine)
Sulfacetamide
Sulfamethozypyridazine (Kynex)

Antimalarials

Primaquine
Pamaquine
Pentaquine
Quinocide
Quinacrine (Atabrine)

Non-Sulfonamide Antibacterial Agents

Furazolidine
Furmethanol
Nitrofurantoin (Furadantin)
Nitrofurazone
Chloramphenicol
Aminoglycoside antibiotics,
i.e., Streptomycin, Gentomicin,
Tobramycin
Metronidazole (Flagyl)

Miscellaneous

Napthalene
Trinitrotoluene
Methylene blue
Naldixic acid
Phenylhydrazine
Quinine

Quinidine
Ascorbic acid
Niridazole

An ETG system can be designed for identification and/or quantification of a selected cell type using the lectin-serum albumin-luminescer conjugate. All that is necessary is to select a lectin with particular affinity for a carbohydrate which is present on the surface of the desired target cell. For instance, a conjugate including the lectin concanavalin A, which has a particular affinity for red blood cells and T cell lymphocytes, serum albumin and a luminescer can be cross-linked with a sample suspected of containing these cell types. The labeled cells, if present will luminesce upon the addition of a base, the amount of chemiluminescence being proportional to the number of cells of these types present. If it is desired to detect or quantify only the red blood cells, the luminescent reaction can be triggered by heat. To test for the presence or number of T cells or the number of both T cells and red blood cells, the lectin-serum albumin-luminescer conjugate is cross-linked in the ETG with a heme protein so that the intensity of the thermochemiluminescent light emitted will be proportional to the number of cells present.

An ETG containing cross-linked heme protein and the luminescer-serum albumin complex can also be used as a thermal dosimeter. The gel can be applied to an insoluble support matrix or contained within a tube or vial. All that is needed to initiate the chemiluminninescent reaction is a source of heat, and the

amount of light emitted is directly proportional to the amount of heat to which the gel is exposed.

The serum albumin-luminescer complex can also be used directly to detect and quantify peroxidative injury to cells. Peroxidative injury may result from incident ionizing radiation, hyperthermia, the presence of autoxidizable compounds or peroxidative and oxidative enzymes, transitory ischemia or inflammation. As a result of exposure to such stimuli, aldehyde groups form on the surface of cells as the product of glycoprotein and unsaturated fatty acid peroxidation. The serum albumin of the soluble serum albumin-luminescer conjugate may be attached to the aldehyde groups using the Schiff base reaction. Excess soluble conjugate which does not bind to the cells is washed away, and when contacted with a base, the cell-bound luminescer is oxidized by the albumin. The resultant luminescence is proportional to the number of surface aldehyde groups that were formed during exposure to the peroxidizing stimulus. If the serum albumin-luminescer complex is coupled with a heme protein and a polyamino compound and that conjugate is attached to the aldehyde groups on the surface of the damaged cells, the luminescent reaction is triggered by heat. For ease in conducting the assay, the cells can be attached to an insoluble support matrix. For instance, red blood cells suspended in appropriate solvents can be air-dried onto a glass slide or attached to beads or other supports in a column and the conjugate and reducing agent necessary for the Schiff base

reaction are added. Unbound conjugate is washed away by adding more solvent, and the light-emitting reaction is triggered by adding base or heating, as the case may be.

This procedure can be modified for use in measuring peroxidative injury to cell types besides red blood cells, in particular, macrophages, monocytes, granulocytes, tumor cells, and primary and established cell culture lines. It may also be modified so that it can be used to establish the sensitivity of cell membranes of both tumor and normal cells to oxygen-dependent antineoplastic pharmaceuticals such as bleomycin and the ICCOPS system described in co-pending application Serial No. 251,694.

An antigen, antibody, or other proteinaceous molecule may also be conjugated to the serum albumin of the serum albumin-luminescer complex. Such a conjugate can be used in an assay to detect and quantify a specific analyte, or ligand, when that specific analyte is a member of a specific binding pair consisting of the ligand and its antiligand. The binding pair can be a substrate and an enzyme specific for that substrate or it can be an antigen and an antibody specific for that antigen. A specific enzyme is conjugated by a bifunctional crosslinking agent to the serum albumin-luminescer complex, and a solution suspected of containing the substrate which that enzyme metabolizes is mixed with the enzyme-serum albumin-luminescer conjugate. The enzyme transfers reducing equivalents from its substrate to oxygen, producing peroxide, superoxide and hydroxyl free radicals which oxidize the luminescer. The amount of

luminescence which results under basic conditions will be directly proportional to the amount of that specific substrate present in the solution. Of course, the serum albumin-luminescer compex can be coupled with substrate rather than the enzyme.

This method is of particular utility when used with a dehydrogenase. For instance, if it is desired to quantify the amount of lactic acid present in the blood, lactate dehydrogenase (LDH) is used. An oxidizable nucleotide cofactor is also necessary. The cofactor can be nicotinamide adenine dinucleotide (NAD) in the case of LDH, or nicotinamide adenine dinucleotide phosphate (NADP), flavin adenine dinucleotide (FAD) or flavin mononucleotide (FMN) depending upon the particular dehydrogenase being utilized.

The luminescent reaction can also be triggered by heat if the enzyme (or substrate) is coupled with a heme protein-serum albumin-luminescer conjugate. When a heme protein is used, the enzyme transfers reducing equivalents directly to oxygen as well as to the heme protein. The serum albumin-luminescer complex can also be conjugated to an antigen or an antibody which, in the presence of the corresponding member of the antigen/antibody binding pair, the cross-linking agent and a base, will luminesce. Viral antigens such as Hepatitis B antigen or bacterial antigens can be detected by conjugating the corresponding antigen with its respective antibody-serum albumin-luminescer conjugate. A heme protein can be covalently bonded to the antibody/antigen-serum

albumin-luminescer conjugate and the light emitting reaction triggered by heat rather than the base.

The serum albumin-luminescer complex can also be conjugated with protein A to form a "universal reagent" for use in many assays dependent on an antibody-antigen binding reaction. Protein A is isolated from the cell walls of Staphylococcus aureus, and has been shown to bind specifically to the Fc region of several immunoglobulins without inhibiting antigen-antibody binding. Because of the ability of protein A to bind to such a large number of immunoglobulins, the protein A-serum albumin-luminescer conjugate can be used as a universal reagent for the formation of a conjugate with an antibody to be used in a chemiluminescent immunoassay as described above for the presence of a particular antigen, for instance, bacterial or Hepatitis B antigens. The antibody-protein A-serum albumin-luminescer conjugate can also be coupled to a heme protein such that the reaction can be triggered by heat rather than base.

The protein A-serum albumin-luminescer conjugate can be used to detect bacteria such as Salmonella sp. in food samples by applying a sample of the foodstuff to a glass slide or to the inside of a tube or vial, and then incubating the sample-coated insoluble support with an antiserum containing an IgG for the appropriate bacterium. The protein A-serum albumin-luminescer conjugate is then added, unbound conjugate is washed away, and the chemiluminescent reaction triggered with a base. In the case of bacteria such as Salmonella newport, S. anatum or S.

typhimurium, an IgG containing antiserum for the appropriate Salmonella H antigen (i.e., i, Phase 1 for S. typhimurium or e,h, Phase 1 for S. newport or S. anatum) is used when the sample is fixed to the insoluble support with formalin. Since heating, acid, and alcohol destroy the H antigen, the sample is fixed using one of these agents to reveal the O antigen, which can then be detected with the appropriate antiserum.

Positive controls containing the salmonella cells with the appropriate antigens can be used. These cells can also be used to detect specific antibody in unknown sera to H and O antigens using the same techniques as above. When heterologous antiserum that contains antibody to several antigens is used, the appropriate H or O antigens can be added along with the antiserum to block the antibodies so as to enable the identification of the specific antigens present in the sample. Bacteria other than Salmonella sp. and the appropriate antisera or monoclonal antibody to flagellar, somatic, capsular or membrane antigens may be substituted into the above method to design specific immunoassays for many other bacteria. Competitive binding assays can also be conducted using these methods.

A convenient assay for antigens such as Hepatitis B antigen can also be conducted using the protein A-serum albumin-luminescer conjugate. Filter paper, or a cellulose acetate disk, is impregnated with protein A and serum albumin, crosslinked, then coated with an antibody specific for Hepatitis B antigen and freeze-dried for storage. When the assay is to be

run, serum or plasma is added to the paper or disk along with the protein A-serum albumin-luminescer conjugate and unbound antiserum and protein A-serum albumin-luminescer is washed away. The amount of chemiluminescence generated upon the addition of base will be proportional to the amount of Hepatitis B antigen present.

In any of the assays involving the binding of a ligand to an antiligand, either the ligand or the antiligand being a part of the conjugate comprised of ligand/antiligand-serum albumin-luminescer, it is important that only the conjugate which is bound to the ligand/antiligand desired to be detected luminesce. A convenient method by which only bound conjugate is made to luminesce is to conduct the assay so that it involves a change in phase when binding of ligand to antiligand occurs. This change in phase may be accomplished by precipitation or agglutination of the bound ligand-antiligand complex or by immobilizing either the ligand or the antiligand. For instance, in the case of the above-described immunological assays, a column is loaded with Sepharose A (a polysaccharide to which protein A is attached) beads (Pharmacia), and an antibody which is specific for a determinant on the antigen desired to be detected is added. The antibody is bound to the beads (i.e., is an immobilized ligand), a fluid sample containing the antigen (the antiligand) is added, and the antigen binds to the immobilized antibody. An antibody-protein A-serum albumin-luminescer conjugate is then added to the column, the antibody of the conjugate being selected

for its specificity for a second determinant on the bound antigen such that the conjugate binds with the bound antigen. Unbound conjugate is then washed through the column, the fractions are eluted and the light-generating reaction triggered. The assay could also be conducted by immobilizing the antigen, i.e., by complexing it with a large protein and crosslinking it in a gel. The same technique can be used to immobilize an enzyme (or the substrate for that enzyme) or a cell type (or the lectin which preferentially binds to that cell type).

The chemiluminescent reaction of the present invention is also used to detect sensitivity to certain immunostimulants. Peripheral lymphocytes and other monocytic cells are collected from the patient. The antigen and serum albumin-luminescer complex are conjugated, unbound conjugate is washed away, and the chemiluminescent reaction triggered by a base added to the washed cells to enumerate the receptors to the conjugate. Spontaneous luminescence of the cells in the presence of the conjugate is used to determine the metabolic response of these immuno-responsive cells. The magnitude of the chemiluminescent reaction is proportional to the cellular immune response of the cells.

Lectins, antigenic proteins (such as serum proteins like immunoglobulins), bacterial products (such as Protein A from Staphylococcus aureus, mycobacterial proteins, or lipopolysaccharides), viral antigens (rabies, measles, hepatitis, herpes, distemper, canine and feline parvoviruses, or human T

cell leukemia virus), or fungal antigens (Histoplasma capsulatum, Coccidiodes immitis, blastomyces, or Cryptococcus neoformans) may be conjugated to the serum albumin-luminescer complex to create specific chemiluminescent cellular immunostimulators.

The conjugation is accomplished by a variety of methods depending upon the nature of the molecule to be conjugated to the serum albumin-luminescer complex. Glutaraldehyde is used to conjugate the serum albumin-luminescer complex to immunostimulators comprised in part of accessible amino groups. For materials such as glycoproteins or lipopolysaccharides, conjugation is accomplished by oxidation of the carbohydrate moiety by periodate. Protein A and other proteins can also be conjugated to the serum albumin-luminescer complex using N-succinimydyl-3-(2-pyridyldithio) propionate.

Another use of the serum albumin-luminescer complex in the context of an immunoassay is to measure the potential for allograft rejection in organ transplant cases. Whole blood is collected from both donor and recipient and the white cells are separated. A sample of donor cells is treated with an agent to arrest cellular proliferation. Donor lymphocytes are mixed with intact recipient lymphocytes in medium containing tritiated thymidine. If the recipient cells are synthesizing DNA, tritiated thymidine will be incorporated in the newly synthesized DNA. The cells are then harvested onto filter paper and the serum albumin-luminescer complex added, along with a bifunctional cross-linking agent. Donor cells cannot chemiluminesce, but the

recipient cells can since they are the only cells that can incorporate radioactive thymidine, and the amount of luminescence will be proportional to the amount of ($^3H$)-thymidine taken up by the recipient cells. The amount of light emitted will be proportional to the immune response of the recipient cells to the donor cells.

The results of this assay are enhanced by virtue of the fact that the tritiated cells will fluoresce (even without liquid scintillation cocktail) and chemiluminesce. Not only is the amount of light emitted enhanced because it is the sum of the fluorescence and the chemiluminescence, but because no liquid scintillation cocktail is necessary, all the components of the assay are kept in close proximity on the filter paper, thereby increasing the efficiency of the light-emitting reaction as compared to fluorescence alone. Similar radio-labeling, harvesting and counting techniques are used for cellular components labeled with $^{14}C$, $^{24}Na$, $^{32}P$, $^{35}S$, $^{40}K$ and $^{45}Ca$.

The serum albumin-luminescer conjugate is also used for autoradiographic enhancement by the following method. Tritiated spleen cells are fixed in an even, thin layer on a glass slide and then contacted with the serum albumin-luminescer complex and a biofunctional cross-linking agent. The slide is then coated with photographic film emulsion and the exposure allowed to continue for a period of time, along with appropriate control slides. The resulting autoradiogram will be enhanced over conventional autoradiograms because the film is exposed to the

conventional direct ionizing radiation effects, aminophthalate fluorescence and chemiluminescence. Further, resonant energy transfer between the luminescer and aminophthlate occurs due to the proximity of these molecules in the hydrophobic binding sites of the cross-linked serum albumin, resulting in increased quantum yield. The transfer efficiency is dependent upon the state (degree of collapse) of the gel which is, in turn, controlled by the solvent content (ratio of protic to aprotic solvents), temperature, and any externally applied electric fields.

This procedure may be adapted to autoradiography of peripheral leukocytes, tissue slices, tissue sections, tumor cells, primary cell lines, and established cell lines. The autoradiography may be applied to biological components (i.e. proteins, carbohydrates, lipids, hormones, cell membranes, or cellular organelles) labeled with $^{51}Cr$, $^{35}S$, $^{14}C$, $^{3}H$, $^{24}Na$, $^{32}P$, $^{40}K$, $^{45}Ca$, $^{131}I$, or other radioisotopes. It may be applied to radiolabeled electrophoretic polyacrylamide techniques or solid support radioimmunoassay techniques utilizing radiographic film. The autoradiography may be quantified by counting silver grains or by performing film densitometry on film contacting but not attached to, the sample. This latter technique is used in solid support radioimmunoassay or radiolabeled electrophoretic polyacrylamide gel techniques.

Another assay which can be conducted using the serum albumin-luminescer complex according to the present invention is to measure the NADH or NADPH content of a sample. Among other

things, NADH and NADPH content can be used as a measure of the number of previously viable cells present, for instance, in a food sample. Measurement of the number of previously viable cells present is made possible because, for instance, in the case of bacterial cells, a relatively stable ratio of NADH to NAD is maintained under a given set of conditions. After the death of the bacterium, NADH is rapidly converted to NAD, so the amount of NADH present is an accurate indicator of the number of viable cells present in a sample.

NADH content is assayed by cross-linking an NADH-dependent methemoglobin reductase, cytochrome $b_5$, hemoglobin and the serum albumin-luminescer complex with a bifunctional cross-linking agent to make an ETG, or by adding iron ethylenediame tetracetic acid (EDTA) in place of incorporating cytochrome $b_5$. A standard curve for the thermochemiluminescence of the gel is prepared by heating the gel and recording the amount of light emitted at each temperature, then recording the same data for gels containing a known amount of NADH, the increase in the amount of light emitted being proportional to NADH concentration. The baseline standard is used to determine the equivalence of multiple batches of gel. To assay NADH content, the sample is treated to lyse the cells and then the sample is added to the ETG.

The number of previously viable eukaryotic cells present in a sample can be determined by substituting an NADPH-dependent methemoglobin reductase for the NADH-dependent methemoglobin reductase since the concentration of NADPH is much higher in the

cytoplasm of viable eukaryotic cells than the concentration of NADH. This preparation lacks the cytochrome $b_5$, but contains flavin adenine dinucleotide (FAD) or flavin mononucleotide (FMN) entrapped in the ETG. Red blood cell hemolysates (with the appropriate protein content) can be substituted for the methemoglobin reductase and hemoglobin in either the assay for viable bacterial or eukaryotic cells.

The present invention can be better understood by reference to the following non-limiting examples.

## EXAMPLE 1. DETECTION OF HEAT

An ETG system was prepared by mixing 10 mg of hemoglobin (Sigma Chemical Co., St. Louis, Mo.), 300 mg of bovine serum albumin (Sigma), and 10 mg of luminol (Sigma) in 10 ml of pH 6.9 phosphate buffered saline (PBS) and filtering the solution through a 0.22 micron bacterial filter (Millipore) to remove undissolved luminol. To this solution, 110 µl of 25% glutaraldehyde (Sigma) is added. The solution was allowed to gel in the refrigerator and then ground in a 1:10 proportion of gel to PBS at pH 7.4. One ml of the gel suspension was then placed in a vial, heated in a water bath and immediately placed in a Beckman LS230 scintillation counter in the out-of-coincidence mode. The results are shown in Fig. 1.

EXAMPLE 2. THERMOCHEMILUMINESCENT ASSAY FOR DETECTION OF MALIGNANT HYPERTHERMIA

Red blood cells (RBCs) were obtained from Landrace-Duroc crossbred pigs with a herd history of malignant hyperthermia, Poland-China pigs inbred for malignant hyperthermia, Sprague-Dawley rats, and a normal human. Freshly drawn RBCs (containing sodium citrate as an anticoagulant) were washed free of plasma and suspended in pH 6.9 0.1 M PBS with 0.154 NaCl containing 0.5% (V/V) glutaraldehyde (Sigma). The cross-linking with glutaraldehyde proceeded for two hours at 4° C. The cells were washed free of unbound glutaraldehyde, centrifuged at 600 x g, and resuspended in pH 6.9 PBS containing 1 mg/ml horseradish peroxidase (HRP, Sigma Type IV) and 1 mg/ml BSA (Sigma) with non-covalently bound luminol (Sigma). This preparation was incubated overnight at 4° C. The cells were washed and stored in pH 7.4 PBS containing 0.5% D-glucose.

To trigger the light-emitting reaction, the cells were heated in a water bath to the desired temperature ± 0.2° C within the range of 30 to 55° C and immediately counted in a Beckman LS 230 liquid scintillation counter set in the out-of-coincidence mode. The results are shown in Fig. 2. Each point represents the mean of three samples of 1 x $10^9$ cells each. The standard error of the instrument was ± 2%. The closed circles represent Landrace-Duroc crossbred porcine cells, the open circles represent miniature porcine cells, open squares represent

Sprague-Dawley rat cells, and closed squares represent human RBCs. The solid line is the linear regression for the Landrace-Duroc crossbred procine cells (correlation coefficient r = 0.979), the line of alternating dots and dashes is the rat data regression (r = 0.940),and the single dashed line is the regression line for combined human and miniature swine data (r = 0.959, pooled since they were indistinguishable).

Fig. 3 shows the thermochemiluminescence of RBCs from Poland-China pigs. The open circles represent nine samples of 3 x $10^8$ cells labeled with luminol, BSA and HRP, the closed circles represent nine samples of 3 x $10^8$ cells coated with BSA and HRP, and the open squares represent nine samples of 3 x $10^8$ cells cross-linked with glutaraldehyde. The mean standard deviation for luminol-labeled RBCs from Poland-China pigs was ± 12.1% with a range of 9.3 to 17.1%, for cells treated with glutaraldehyde only, the standard deviation was ± 10.6 percent, ranging from 6.6 to 20.4%; and for BSA and HRP coated cells, the standard deviation was ± 14.7% with a range from 10.8 to 18.7%.

To control for luminescence originating from the reagents independently of the RBC's, 1 mg HRP/ml and 1 mg BSA with luminol/ml of pH 7.4 PBS were heated and the luminescence measured. The thermochemiluminescence of these reagents was at background levels.

## EXAMPLE 3. TESTING FOR GLUTATHIONE DEFICIENCY

Freshly drawn human red blood cells (RBCs) were cross-linked with glutaraldehyde, horseradish peroxidase, BSA and luminol in the same manner as set forth in Example 2, above. Multiple samples of human RBCs were pretreated with 1-chloro-2,4-dinitrobenzene (CDNB) prior to luminescent labeling to deplete endogenous glutathione. This depletion was accomplished by incubating the samples in pH 7.4 PBS containing 0.5 mM CDNB in a water bath for fifteen minutes at 37° C. The cells were then washed and resuspended in fresh pH 6.9 PBS for labeling of cells with the HRP, luminol and BSA as previously described. To test the effects of the metabolic stressor halothane (2-bromo-2-chloro-1:1:1-trifluoroethane), 20 µl of halothane were added to a 1.0 ml sample of both normal and CDNB-treated RBCs prior to induction of thermochemiluminescence. The scintillation vials were sealed to prevent escape of the volatile halothane. The samples were heated in a water bath to the desired temperature ± 0.2°C within the range of 30 to 50°C and immediately the amount of light emitted was measured with a Beckman LS 230 liquid scintillation counter set in the out-of-coincidence mode. The results are shown in Fig. 4. Each data point represents the mean of three 3 X $10^8$ cell samples. The closed squares represent the data for normal human RBCs, the open circles represent normal human RBCs that were exposed to

halothane, the closed circles represent RBCs that were treated with CDNB, and the open squares represent the data from CDNB-treated RBCs that were exposed to halothane.

The effect of halothane on the thermochemiluminescence of Landrace-Duroc RBCs obtained from a herd with a history of malignant hyperthermia was also tested. Cells were prepared by the same method as above, and 20 µl of halothane was added to 1 ml cell samples containing 1 X $10^9$ cells. The results are shown in Fig. 5. The open circles represent data from untreated RBCs; the closed squares represent porcine RBCs stressed with halothane.

## EXAMPLE 4. VERIFICATION OF THERMOCHEMILUMINESCENT ASSAY AS INDICATOR OF AUTOXIDATIVE DEFICIENCIES

In order to help verify that the thermochemiluminescent assay was actually acting as an indicator of autoxidative deficiencies, inhibition studies were conducted with the rat RBCs. The RBCs were drawn and prepared as described in Examples 2 and 3, above. Superoxide dismutase (Sigma), catalase (Sigma), copper sulfate (Fisher) or sodium nitrite (Fisher) was then added to a 1 ml cell suspension of labeled rat RBCs (1 x $10^8$ cells) in pH 7.4 PBS. Five sets of three 1 ml samples each were prepared. One set of 3 samples received 5 µg of superoxide dismutase, one set received 60 µg of catalase, one received 9.0 µM of copper sulfate and one set of samples received 33.0 µM of sodium nitrite. The enzymes remained in solution during the thermochemiluminescent assay. The cells were washed free of copper sulfate or sodium

nitrite by centrifugation and resuspension after thirty minutes of pre-incubation prior to the thermochemiluminescent assay. The cells were heated to 50°C before measuring chemiluminescence. The following table demonstrates the inhibition of chemiluminescence by superoxide dismutase, catalase, copper sulfate and sodium nitrite (each number represents the mean of three samples):

| Reagent | CPM ± S.D.* (N = 3) | Percent Activity |
|---|---|---|
| None | 149,519 ± 2777 | 100 |
| Superoxide dismutase | 49,476 ± 6958 | 33.8 |
| Catalase | 46,566 ± 9123 | 31.28 |
| $CuSO_4$ | 34,145 ± 2691 | 23.3 |
| $NaNO_2$ | 31,182 ± 2983 | 21.3 |

*S.D. = standard deviation; the cpm of the empty vial was 17,905 ± 4662 (N=3).

Inhibition of the chemiluminescent reaction by superoxide dismutase and catalase indicates the participation of superoxide and hydrogen peroxide, respectively, in the reaction. Copper sulfate and sodium nitrite, which convert oxyhemoglobin to methemoglobin, also inhibited the reaction, indicating the participation of oxyhemoglobin in the autoxidative reaction.

EXAMPLE 5. MEASUREMENT OF PEROXIDATIVE INJURY TO CELLS

The extent of peroxidative injury suffered by cells may be assayed in two ways. In the assay, 1 ml of fresh heparinized blood from a human or other animal previously exposed to a peroxidizing agent is centrifuged at 400 x g for fifteen minutes and resuspended in ten times the cell volume of pH 6.9 PBS. This washing is then repeated and the cells are centrifuged again and resuspended in 1 mg of BSA saturated with absorbed luminol in pH 6.9 PBS. The cells are incubated in this preparation for two hours at 4° C, then washed three times in pH 7.4 PBS. The cells are then resuspended in pH 7.4 PBS containing 2 mM potassium borohydride and incubated for thirty minutes on ice, then washed three times in pH 7.4 PBS.

The amount of chemiluminescence is determined by adding 0.1 ml of 0.1 N NaOH to 0.5 ml of RBCs (diluted 1:20) and measuring the light emitted in a luminometer or scintillation counter set in the out-of-coincidence mode.

A negative control can be made by pretreating the cells with the potassium borohydride prior to contacting them with bovine serum albumin and luminol. A positive control can be made by peroxidizing the cells with sodium periodate. The cells are treated with 1.5 mM $NaIO_4$ dissolved in pH 7.4 PBS. The process is allowed to continue for fifteen minutes on ice and the cells

are then washed three times in pH 7.4 PBS and treated with the BSA and luminol as previously described.

The second method for conducting this assay involves the substitution of a hemoglobin-BSA-luminol conjugate for the BSA-luminol complex described above. This complex is prepared by mixing 2 mg/ml of hemoglobin with 2 mg/ml BSA, 1 mg/ml of luminol, and 5 µl/ml of 25% glutaraldehyde in pH 6.9 PBS. This mixture is then passed through a 0.22 µ bacterial filter (Millipore) and incubated at 4° C for one hour. After incubation, the cross-linking reaction is stopped by the addition of 100 mg of the polyamino compound lysine. The sample is loaded into a G-25 Sephadex (Pharmacia) column (48 ml) and collected in 4 ml fractions. The fractions are tested for thermochemiluminescence by heating to 50° C and immediately measuring the luminescence in a luminometer or beta liquid scintillation counter in the out-of-coincidence mode. The luminescent reaction may also be triggered by adding base, base and hydrogen peroxide, or by heating to any temperature greater than 42° C.

EXAMPLE 6. IDENTIFICATION AND QUANTIFICATION OF SPECIFIC CELL TYPES

Red blood cells were labeled for identification as follows. The labeling reagent was prepared by mixing 0.2 ml of 2 mg/ml of the lectin concanavalin A (Con A) with 1.6 ml of pH 6.9 PBS, 0.2 ml of 2

mg/ml BSA, 10 µl of 25% glutaraldehyde, and 2 mg of luminol. This preparation was passed through a 0.22 µ filter (Millipore) and incubated in the refrigerator for one hour. After incubation, the reaction was stopped by the addition of 100 mg of glycine. The cross-linked sample was loaded on a G-25 Sephadex (Pharmacia) column (48 ml) equilibrated in pH 7.4 PBS. Four milliliter fractions were collected from the column, and 0.5 ml samples from each 4 ml fraction were added to 1 ml of 1:10 human RBCs in pH 7.4 PBS. This preparation was incubated for fifteen minutes at 37° C. The cells were then washed three times with pH 7.4 PBS and resuspended in 2 ml of pH 7.4 PBS.

The chemiluminescent reaction was activated by adding 100 µl of 0.1 N NaOH to 0.5 ml of cell suspension, and the light reaction counted in the luminometer at a setting of 6. The counts per minute (CPM) from the luminometer at a setting of 6 were equivalent to $10^{-3}$ X CPM from a Beckman LS230 scintillation counter set in the out-of-coincidence mode. The results are presented graphically in Fig. 6.

EXAMPLE 7. MEASURING DRUG SENSITIVITY

The sensitivity of an individual to certain toxic compounds is measured by preparing a Con A-BSA-luminal conjugate according to the method of Example 6, above and then coating RBCs from an individual suspected of sensitivity to that compound with the conjugate by the same method as that disclosed in Example 6. The RBC samples coated with the conjugate are heated to a temperature from between 25 to 50°

C in the presence, and then in the absence, of the toxic compound, and the luminescence is measured. Excess luminescence, being a result of excess superoxide, hydrogen peroxide and hydroxyl free radical production, which causes the oxidation of the luminol, is an indication of sensitivity.

EXAMPLE 8. CELL-MEDIATED ANAMNESTIC RESPONSE OF MURINE SPLEEN CELLS

A luminol mixture for immunizations was prepared by suspending 3 mg/ml luminol (Baker Chemical Co., Phillipsburg, N.J.) and dissolving 3 mg/ml bovine serum albumin (BSA) (Sigma) in phosphate buffered saline (PBS), pH 7.4. A luminol indicator for the chemiluminescent assay was prepared by making a 1:5 dilution of a freshly prepared solution of 1 mg/ml of luminol and 1 mg/ml BSA in PBS, pH 6.9. Both luminol mixutures were prepared at room temperature, filtered through a 0.2 µm Gelman Acrodisc to remove undissolved luminol, and stored at 4° C in the dark until use.

Male CBA/J or BALB/cj mice (Jackson Laboratories, Bar Harbor, ME.), 25 - 30 g, were maintained on a 12/12 light/dark cycle with food and water ad libitum. Control animals were immunized with 0.5 ml saline (subcutaneous or intraperitoneal) and experimental animals received 0.2 ml of the luminol-BSA antigen subcutaneously.

At two to four days after immunization, the mice were killed by rapid cervical dislocation and their spleens were removed. The spleens were teased apart in room temperature Hank's Balanced

Salt Solution (HBSS) buffered with 20mM HEPES (N-2 hydroxyethylpiperazine-N'-2-ethanesulfonic acid, Sigma), pH 7.4, 20 mg spleen/ml HBSS. The suspension was centrifuged at 6 x g for 3 minutes to remove tissue debris. Cell viabilities were determined by exclusion of trypan blue dye, and the cell concentration was adjusted to 8 - 12 X $10^3$ viable cells/$mm^3$. Differential counts made of stained fixed slides showed the suspension to be 94-96% lymphocytes.

After incubation of 1 ml samples in dark-adapted glass scintillation vials for 25 minutes in a 37° C shaker water bath, 100 µl of the luminol-BSA antigen mixture was added to each vial. Chemiluminescence was measured before, at, and every 5 minutes after the addition of luminol on a Beckman Model LS230 liquid scintillation counter set in the out of coincidence mode (gain 350, preset 0.2 min., window 0-100). Cells were maintained at 37° C between readings.

The chemiluminescent response of the splenocytes to <u>in vitro</u> primary and secondary exposure to antigen was as follows:

| Group | Total Chemiluminescence (cpm X $10^3$) | Average Chemiluminescence (cpm X $10^3$) |
|---|---|---|
| Control | 169.4 ± 68.7 | 24.2 ± 25.9 |
| 2 Day | 484.7 ± 155.6 | 69.2 ± 58.8 |
| 4 Day | 1405.8 ± 439.8 | 200.8 ± 166.2 |

Because no differences were observed in the responses of the two strains of mice, the data were pooled. The total chemiluminescence represents the cpm above background from 5 minutes after addition of luminol until 35 minutes after, and the average chemiluminescence is the mean of all measurements from 5 until 35 minutes after addition of luminol. Actual results are presented graphically in Fig. 7. Closed squares represent data from the Control group, open circles represent data from the 2 Day group, and open squares represent data from the 4 Day group. The Control group consisted of four mice, the 2 Day and 4 Day groups were three mice each, and 7 measurements were made on each mouse. The mean of the 4 Day group differs from the mean of both the Control and 2 Day groups; $p < 0.01$ for Duncan's multiple range test.

EXAMPLE 9. MEASUREMENT OF POTENTIAL FOR ALLOGRAFT REJECTION IN TRANSPLANT CASES

Collect 10 ml of whole, heparinized blood from both donor and recipient. Add 4 ml of Ficoll-Hypaque to each 16 X 105 mm polycarbonate tube and gently layer 2-6 ml of blood over the Ficoll-Hypaque. Centrifuge at 2000 x g for 20 minutes. Collect all the white cells in the fluid above the red cell pellet. Add 10 ml of balanced salt solution containing 5% fetal calf serum (FCS), and centrifuge at 300-350 x g for 15 minutes. Wash twice and centrifuge at 250 x g for 10 minutes. Count cells with a Coulter cell counter and adjust the cell concentration to 1 - 6 X $10^7$ cells/ml. Add 25 µg of mitomycin C (50 microliters of 0.5 mg/ml membrane-filtered, sterilized mitomycin C solution) in balanced salt solution to each ml of cell suspension. Incubate this preparation for 20 minutes at 37° C in the dark. Wash 3 times, as before, in balanced salt solution containing 5% FCS. Adjust the concentration of untreated responder cells and the inhibited stimulator and responder cells to 2 X $10^6$ cells/ml in RPMI 1640 medium supplemented with L-glutamine, sodium pyruvate, nonessential amino acids, and 5% FCS. For control cultures, mix 0.5 ml of uninhibited responder cells with 0.5 ml of inhibited responder cells in a 12 X 75 mm culture tube. For test cultures, mix 0.5 ml of untreated responder cells with 0.5 ml of treated stimulation cells.

Place the tubes in a slant tube rack and incubate in a 37° C humidified incubator in an atmosphere of 5% $CO_2$ in air. Pulse

each culture with 0.1 ml of tritiated ($^3$H)-thymidine (1-2 microcuries) for 72 - 96 hours.

Harvest the cells as follows: a separate Millipore filter (0.45 micrometer) held in a suction filtration device is used to remove cellular material from each culture suspension after vortexing. Two milliliters of cold balanced salt solution are added to each culture tube, which is then vortexed. The contents are poured onto the filter. This process is repeated. The cells are then washed on the filter with 10 ml of cold 10% trichloroacetic acid two times in succession. The filter is then washed with 2 ml 95% ethanol or absolute methanol 3 times in succession.

An aqueous solution is prepared of 30 mg/ml of BSA saturated with luminol (previously filtered with a 0.22 µm filter to remove insoluble luminol) and is adjusted to pH 6.9. To this solution is added 20 microliters of 25% glutaraldehyde per ml. The solution is passed through the filter for 10 minutes or until the flow ratio decreases by at least 50%. This filter is again dehydrated with absolute methanol, dried, and counted in a beta scintillation counter. Standards containing a known amount of radioisotope material ($^3$H-labeled) on the disk must be prepared to determine the counting efficiency and quenching.

EXAMPLE 10. AUTORADIOGRAPHIC ENHANCEMENT

Spleen cells are labeled by ($^3$H)-thymidine (10 µCi/ml of culture medium) by incubation for approximately 6 hours at 37° C on a rocker in a humidified cell culture incubator. The cells are removed from the culture dish into a test tube and washed twice in 1 mM unlabeled thymidine. The cells are centrifuged and resuspended in Hank's balanced salt solution to a concentration of 1 - 2 X $10^6$ cells/ml. In the middle of a 0.5% agarose coated slide is placed 0.1 ml of cell sample. To this sample is added 0.1 ml of 2% acetic acid. The sample and acetic acid are spread in a thin layer together by rolling a small glass rod over the gel surface. The slide is dried by several rapid passages through a low flame. After drying and cooling, the slide is dipped 20 times in distilled water to remove salt. The slide is then covered with 1 mg/ml of the BSA-luminol complex in distilled water. The sample is dried in a low flame as before. The slide is then covered with 0.5% glutaraldehyde and allowed to fix for 30 minutes at 4° C. The slide is again washed in 4° C distilled water. After warming to room temperature, the slide is coated with film emulsion (NTB-2 nuclear track emulsion, Eastman Kodak) under a safe light by dipping using a standard procedure (see J. Watson, Autoradiography of Spleen Cell Cultures, in Selected Methods in Cellular Immunology, B. Mishell and S.M. Shiigi, Eds., San Francisco: W.H. Freeman and Co., 1980, pp. 166-172).

The autoradiogram exposure is allowed to proceed no longer than 7 days. Several control slides should be developed over the

exposure time using standard methods (D-19 developer from Eastman Kodak) at 16° C for 3 - 5 minutes and a Rapid Fix for 8 - 10 minutes.

EXAMPLE 11. OXIDATIVE RESPONSE OF MURINE SPLENOCYTES TO SPECIFIC AND NONSPECIFIC IMMUNOSTIMULATORS

Murine splenocytes were prepared in the same manner as described in Example 8, above, from CBA/J mice immunized with either 0.6 mg of bovine serum albumin (BSA), 0.2 ml of a 2% solution of sheep red blood cells (RBCs), 0.2 ml of complete Freund's adjuvant (CFA), or CFA with BSA. The splenocyte preparations were challenged with BSA to which luminol had been absorbed by the same method described in Example 8.

The maximum amount of chemiluminescence was recorded from the splenocytes of those mice which had been immunized with CFA, with or without BSA. The chemiluminescent reactions of the splenocytes from those mice immunized with BSA and sheep RBCs varied, revealing a combination of specific and nonspecific lymphocytic oxidative responses to the BSA.

EXAMPLE 12. OXIDATIVE RESPONSE OF HUMAN LYMPHOCYTES TO E. COLI ANTIGEN

Peripheral human lymphocytes (and other monocytic cells) are collected by the Ficoll-Hypaque method from 10 ml of freshly collected heparinized whole blood. The cells are resuspended in HEPES-balanced salt solution at a pH of 7.4 to a concentration of

$10^6$ cells/ml, then exposed to E. coli LPS-bovine serum albumin (BSA)-luminol conjugate, and the luminescence recorded as described in Example 8.

The E. coli LPS antigen-BSA-luminol conjugate is formed as follows. A 2 mg/ml solution of E. coli LPS, a lipopolysaccharide from Escherichia coli, diluted in 20 ml of 50 mM acetate buffer, pH 5.6, is mixed with 0.4 ml of periodate solution containing 40 µM $KIO_4$. The solution is stirred for 4 hours at 25° C. The reaction is terminated by adding 0.025 ml ethylene glycol (0.45 mM) and stirring for an additional 30 minutes. The solution is dialyzed against 50 mM acetate buffer, pH 5.59, until no further dialyzable material is present. The dialyzed lipopolysaccharide is mixed with an equal volume of water containing 2 mg/ml of BSA. The solution is adjusted to pH 9 with 0.1 N NaOH and stirred at 25° C for 1 hour. The solution is then adjusted to pH 7.0 with 0.1 N HCL.

To this solution is added 1 mg luminol per ml. The mixture is stirred for 30 minutes and then filtered with a 0.22 µ Millipore filter. The filtrate is passed through a G-25 Sephadex column to remove free luminol. The fractions are assayed by binding to antibody specific for E.coli O antigen (lipopolysaccharide) immobilized on a solid support and then covering the solid support with base (0.1 N NaOH) to activate luminescence. The luminescence is measured with a liquid scintillation counter in the same manner as described above.

EXAMPLE 13. PREPARATION OF UNIVERSAL REAGENT FOR USE IN CHEMILUMINESCENT IMMUNOASSAYS

Protein A is linked to bovine serum albumin (BSA) by a modification of the method described by A. Surolia and D.Pain (Vol. 73, Immunochemical Techniques, J.J. Langone and H. Von Vunakis, Eds., New York: Academic Press, pp. 183-191 (1981)). BSA (2.0 mg) is dissolved in 0.2 ml pH 7.5 PBS and then mixed with 0.2 ml N-succinimidyl-3(2-pyridyldithio)-propionate (SPDP) dissolved in ethanol (700 µg/ml). The SPDP is added in dropwise with stirring, and the solution is occasionally stirred for an additional 30 minutes at 25° C thereafter.

Excess reagents and low molecular weight reaction products are removed by gel filtration on G-25 Sephadex equilibrated in PBS pH 7.5. The fractions are pooled and concentrated to 0.3 ml by ultrafiltration. Protein A is prepared in a similar fashion using 1.42 mg of protein A and 0.06 ml of 700 mg of SPDP per ml in ethanol. After gel filtration on G-25, the fractions are pooled and concentrated to 0.3 ml.

The 2-pyridyl disulfide groups are reduced with dithiothreitol (DTT) at a final concentration of 0.05 M to obtain a thiolated product of BSA. Excess DTT and pyridine-2-thione are removed by G-25 gel filtration. The thiolated BSA and 2-pyridyl disulfide containing protein A are mixed for 20 hours at 25° C. Luminol is then added to the conjugate at 1 mg/ml, filtered as described in Example 12, above, and gel filtered on a G-25 Sephadex column (48 ml).

Fractions are tested for the presence of the protein A-BSA-luminol conjugate by adding 0.2 ml of a given fraction to a solid support containing gamma globulin bound to specific antigen (i.e., peroxidase and antiperoxidase antibody). The fraction is incubated on the solid surface for 30 minutes at 25° C and the unbound materials washed away with pH 7.4 PBS. By covering the solid support with 0.1 N NaOH and measuring the luminescence, the presence of the protein A-BSA-luminol conjugate can be determined in the fraction.

## EXAMPLE 14. USE OF UNIVERSAL REAGENT IN CHEMILUMINESCENT IMMUNOASSAY

Human red blood cells (RBCs) are attached to a microscope slide by either methanol or acid fixing (i.e., 2% acetic acid), both methods being known in the art. Serum suspected of containing antibody to specific antigenic sites on the RBCs is incubated on the surface of the coated slide for between 30 minutes to two hours at room temperature (or overnight at 4° C). The slide is then rinsed with phosphate buffered saline (PBS), pH 7.4, to remove unbound antibody.

The protein A-BSA-luminol conjugate prepared according to the method described in Example 13, above, is then added to the slide surface in a PBS carrying solution, pH 7.4. The slide is incubated for 30 minutes - two hours at room temperature (or overnight at 4° C) and free reagent is washed away with PBS, pH

7.4. The luminescent reaction is then triggered by flooding the slide with 0.1 N NaOH.

EXAMPLE 15. DETECTION OF *SALMONELLA TYPHIMURIUM* IN FOODSTUFFS

A homogenized or liquified food sample is placed on the surface of a glass slide or in a vial or tube. The sample is dried and then fixed with 10% buffered (pH 7.4) formalin. The sample slide is gently washed in pH 7.4 PBS and air dried. The sample on the slide is then covered with a 1:1000 dilution of IgG containing antiserum for the i, Phase 1 H antigen of *S. typhimurium*. The sample is incubated in a humidified chamber at 37° C for 1 hour. The unbound antibody is washed away with pH 7.4 PBS containing 1% BSA. Next, the sample is covered with a solution containing 10 µg/ml of the protein A-BSA-luminol conjugate in pH 7.6 PBS prepared according to the method of Example 13, above. The sample is incubated at 37° C for 30 minutes, and subsequently is washed in pH 7.6 PBS to remove free protein A-BSA-luminol. The slide is air dried. The sample is activated by flooding the surface with 0.1 N NaOH and measuring the chemiluminescence as previously described.

EXAMPLE 16. DETECTION OF HEPATITIS B ANTIGEN

A filter paper or cellulose acetate disk is impregnated with protein A and BSA cross-linked at sufficient concentration with glutaraldehyde to form a gel. Prior to gel formation, the

solution containing the gel components is forced into the filter disk. After gelling, the disk is washed with 1 ml of PBS pH 6.9 containing 100 mg of glycine. The disk is dehydrated and stored in the freezer at -40° C until used. The disk is coated with anti-Hepatitis B antigen antibody when ready for use by incubating the disks in a 1:1000 dilution of the antibody in PBS pH 7.4 overnight at 4° C. The disk is washed by dipping 20 times in PBS, pH 7.4, containing 1% BSA. The disk may be freeze-dried for prolonged storage at -70° C or kept moist at 4° C.

Serum or plasma (0.1 ml) is added to the disk for the detection of Hepatitis B antigen and incubated for 1 hour at 37° C in a humidified chamber. The disk is then washed by dipping 20 times in PBS, pH 7.4. Following this washing, the disk is again incubated with anti-Hepatitis B antigen antibody for 1 hour at 37° C in a humidified chamber. Again, the disk is washed in pH 7.4 PBS. Finally, the protein A-BSA-luminol conjugate (0.1 ml), prepared as described in Example 13 above, is added to the disk and incubated for 30 minutes at 37° C. The disk is again washed free of unbound protein A-BSA-luminol. The chemiluminescent reaction is triggered by adding 0.1 ml of 0.1 N NaOH to the surface of the disk and measuring the light emitted in a beta scintillation counter set in the out-of-coincidence mode.

## EXAMPLE 17. ASSAY FOR ALCOHOL CONTENT

A dehydrogenase luminescent gel for detection of alcohol can be prepared by first coupling $N^6$-[N-(6-aminohexyl)

carbamoylmethyl]-NAD to alcohol dehydrogenase. The method for this coupling is a modification of the method that has been described by M.O. Mansson, PO. Larsson, and K. Mosbach (86 European Journal of Biochemistry, 455-463 (1978)). Briefly, for alcohol dehydrogenase, the method is as follows:

(1) Coupling and purification of product is at 4° C.

(2) Purified horse liver alcohol dehydrogenase (5 mg) is added to 1.3 ml 50 mM triethanolamine buffer, pH 7.5, containing 5 mM $N^6$-[N-(6-aminohexyl) carbanoylmethyl]-NAD.

(3) The conjugating agents 1-ethyl-3 (3-dimethyl-aminopropyl)-carbodiimide hydrochloride and N-hydroxysuccinimide are added to a final concentration of 50 mM and 25 mM, respectively.

(4) The carbodiimide is added in four equal portions at 12-hour intervals and N-hydroxysuccinimide is supplied in two equal portions at 0 and 12 hours.

(5) After a total conjugating time of 48 hours, with intermittent adjustment of the pH to pH 7.5 with NaOH, glycine buffer (pH 7.5) is added to a final concentration of 0.1 M and allowed to react with residual carbodiimide or activated carboxyl groups.

(6) The reaction solution is then dialyzed for 15 hours against three 3-liter changes of 0.05 M glycine in 0.05 M sodium bicarbonate buffer, pH 7.5.

(7) The reaction solution is then applied to a Sephacryl S-200 (Pharmacia) column (1 X 95 cm) equilibrated with the 0.05 M pH 7.5 bicarbonate buffer. The protein-containing fractions are

pooled and dialyzed overnight against 4 liters of bicarbonate buffer.

The alcohol dehydrogenase-NAD complex (0.3 mg) is mixed with 10 mg horseradish peroxidase (HRP) and 30 mg of the BSA-luminol complex. This preparation is prepared by the method of Example 1, above, except that HRP replaces the hemoglobin of the preparation in Example 1. To this solution is added 20 µl of 25% glutaraldehyde per ml.

The solution is absorbed into filter paper (or spread onto plastic or acid cleaned glass slides, coverslips, or the inside of 10 X 50 mm test tubes). The solution is allowed to form the gel coating on the inert support in a humidified chamber at 4° C overnight. The addition of a sample containing ethanol to this gel results in the chemiluminescent reaction, the intensity of the reaction being directly proportional to the alcohol concentration in the sample. A standard curve is plotted by preparing a number of solutions of known concentrations of ethanol, adding those solutions to the gel surface and measuring the light emitted. To do so, amounts of ethanol ranging from 50 µM - 10 mM are added to a solution containing 7 mM lactaldehyde in 0.2 M potassium phosphate buffer, pH 8.0, and each solution is added to the gel surface at 30° C.

EXAMPLE 18. ASSAY FOR VIABLE BACTERIA BY MEASURING NADH CONTENT

Methemoglobin reductase (1 mg/ml) and cytochrome $b_5$ (1 mg/ml) are conjugated with SPDP by a procedure similar to the procedure described in Example 13, above. The only difference in the procedures is that methemoglobin reductase is substituted for the protein A in the procedure described in Example 13. Following G-25 Sephadex gel filtration, fractions containing cytochrome $b_5$ (based on the absorption of light at a wavelength of 405 nm as measured by a spectrophotometer) are pooled. These fractions are concentrated by ultrafiltration to 1 ml of protein solution. This concentrate is added to 9 ml of pH 6.9 PBS containing 30 mg/ml of the BSA-luminol complex and 1 mg/ml hemoglobin. Next, the bifunctional coupling reagent glutaraldehyde (20 µl of 25% glutaraldehyde per ml) is mixed into the solution by stirring. The cytochrome $b_5$ may be replaced with 0.5 micromoles/ml of iron-ethylendiamine tetracetic acid (EDTA) complex added to the BSA-luminol complex at 25° C. The forming gel is used to saturate filter paper, coat glass or plastic slides, or form a free-standing gel. The gel is washed with distilled water by dipping or on a sinter glass filter. The free standing gel is ground with a tissue grinder in 10 times its volume of distilled water. The gel is washed 3 times in distilled water by suspension and centrifugation (300 x g for 10 minutes). By heating the gel from 25 to 50° C and recording the

amount of light emitted at each temperature, then plotting temperature versus luminescence, a standard thermochemiluminescence plot can be generated. This process is repeated with fresh gel in a suspension or coating solution composed of concentrations of NADH ranging from 50 nM to 10mM in pH 7.5 phosphate buffer. The increase in thermochemiluminescence at a given temperature is proportional to the NADH concentration.

To determine the number of viable bacterial cells in a sample, the cells are first lysed with a detergent using methods known in the art, and the solution containing the lysed cell contents added to the gel. The amount of NADH released is directly dependent on the number of viable cells previously present in the sample, therefore the amount of light emitted by the chemiluminescent reaction is proportional to the number of viable cells previously present in the sample.

The above examples are presented for the purpose of illustrating the presently preferred embodiments of the invention, and not by way of limitation.

CLAIMS

1. A device for detecting ionizing radiation or heat comprising:

a luminescer;

serum albumin, said luminescer being non-covalently bonded to said serum albumin to form a luminescer-serum albumin complex;

a bifunctional crosslinking agent; and

an insoluble support matrix, said luminescer-serum, albumin complex being crosslinked in said bifunctional crosslinking agent on said insoluble support matrix.

2. A device for detecting ionizing radiation or heat comprising:

a conjugate comprised of a heme protein, serum albumin and a luminescer, said luminescer being non-covalently bonded to said serum albumin;

a bifunctional crosslinking agent; and

an insoluble support matrix, said conjugate being crosslinked in said bifunctional crosslinking agent on said insoluble support matrix.

3. A device as claimed in claim 2, wherein said conjugate is crosslinked with the bifunctional crosslinking agent to form a gel, said gel being formed on said insoluble support matrix.

4. The device of any of claims 1 to 3, wherein said luminescer is luminol, a luminol derivative or derivatives, phenol pyruvate, indole propionic acid, indole acetic acid, indole-3-pyruvate, or tryptophan, N-acetyltryptophan, or other tryptophan derivatives.

5. The device of any of claims 1 to 4, wherein said bifunctional crosslinking agent is glutaraldehyde, a carbodiimide, succinyl anhydride or N-succinimidyl-3-[2-pyridyldithio]-propionate.

6. The device of any of claims 2 to 5, wherein said heme protein is hemoglobin, myoglobin or a peroxidase.

7. A composition for detection of ionizing radiation or heat comprising a conjugate comprised of a heme protein, a luminescer and serum albumin, said luminescer being non-covalently bonded to said serum albumin, and said conjugate being cross-linked in a bifunctional crosslinking agent to form a gel.

8. The composition of claim 7, wherein said luminescer is luminol, a luminol derivative or derivatives, phenol pyruvate, indole-3-pyruvate, tryptophan, N-acetyltryptophan or other tryptophan derivatives, indole propionic acid or indole acetic acid.

9. The composition of any of claims 7 or 8, wherein said bifunctional crosslinking agent is glutaraldehyde, a carbodiimide, succinyl anhydride or N-succinimidyl-3-[2-pyridyldithio]-propionate.

10. The composition of any of claims 7 to 9, wherein said heme protein is hemoglobin, myoglobin or a peroxidase.

11. The composition of any of claims 7 to 10, wherein said heme protein is bonded to said serum albumin.

12. A composition as claimed in claim 7, comprising a conjugate comprised of hemoglobin, luminol and serum albumin, said luminol being non-covalently bonded to said serum albumin, and said hemoglobin being bonded to said serum albumin, said conjugate being cross-linked in glutaraldehyde to form a gel.

13. A composition as claimed in claim 7, comprising a conjugate comprised of a peroxidase, luminol and serum albumin, said luminol being non-covalently bonded to said serum albumin, and said peroxidase being bonded to said serum albumin, said conjugate being cross-linked in glutaraldehyde to form a gel.

14. A method of detecting ionizing radiation or heat comprising:

admixing a luminescer, a heme protein and serum albumin under conditions promoting the non-covalent bonding of said serum albumin to said luminescer to form a heme

protein-serum albumin-luminescer conjugate;

contacting said conjugate with a bifunctional cross-linking agent to form a gel; and

exposing said gel to a suspected source of ionizing radiation or heat.

15. The method of claim 14, wherein said luminescer is luminol, a luminol derivative or derivatives, phenol pyruvate, indole-3-pyruvate, tryptophan, N-acetyltryptophan or other tryptophan derivatives, indole propionic acid or indole acetic acid.

16. The method of any of claims 14 or 15, wherein said heme protein is hemoglobin,myoglobin or a peroxidase.

17. The method of any of claims 14 to 16, wherein said gel is applied to an insoluble support matrix.

18. The method of any of claims 14 to 17, wherein said bifunctional crosslinking agent is glutaraldehyde, a carbodiimide, succinyl anhydride or N-succinimidyl-3-[2-pyridyldithio]-propionate.

19. The method as claimed in claim 14, in which said gel is formed at temperatures of between approximately 4°C and approximately 25°C.

20. A method as claimed in claim 14, comprising:

admixing luminol, a peroxidase and serum albumin under conditions promoting the binding of said serum albumin to said peroxidase and the non-covalent binding of said serum albumin to said luminol to form a peroxide-serum albumin-luminol conjugate;

contacting said conjugate with glutaraldehyde to form a gel; and

exposing said gel to a suspected source of ionizing radiation or heat.

21. A method of detecting ionizing radiation comprising;

radiolabelling a cell sample;

contacting said cell sample with a non-covalently bonded complex of serum albumin and a luminescer;

contacting said cell sample with a film emulsion; and

thereafter developing a photographic image of said cell sample.

22. The method of claim 21, wherein said cells are labelled with a radiosotope $^{3}H$, $^{14}C$, $^{24}Na$, $^{32}P$, $^{35}S$, $^{40}K$, $^{45}Ca$, $^{51}Cr$ or $^{131}I$.

23. The method of claims 21 or 22, wherein said luminescer is luminol, a luminol derivative or derivatives, phenol pyruvate, indole-3-pyruvate, tryptophan, N-acetyltryptophan or other tryptophan derivates, indole propionic acid or indole acetic acid.

24. The method of any of claims 20 to 23, wherein said bifunctional crosslinking agent is glutaraldehyde, a carbodiimide, succinyl anhydride or N-succinimidyl-3-[2-pyridyldithio]-propionate.

25. A method of measuring peroxidative injury to living cells comprising:

contacting living cells suspected of having suffered peroxidative injury with a complex comprised of a luminescer and serum albumin, said luminescer being non-covalently bonded to said serum albumin;

contacting said cells with a reducing agent;

removing any of said complex which does not bind to said cells;

triggering a light-generating reaction; and

measuring the amount of light emitted.

26. The method of claim 25, wherein said reducing agent is potassium borohydride.

27. The method of claims 25 or 26, wherein said light-generating reaction is triggered by contacting said cells with a base.

28. The method of any of claims 25 to 27, wherein said luminescer is luminol, a luminol derivative or derivatives, phenol pyruvate, indole-3-pyruvate, tryptophan, N-acetyltryptophan or other tryptophan derivatives, indole propionic acid or indole acetic acid.

29. The method of claim 28, wherein said conjugate is soluble in polar solvents.

30. The method of any of claims 28 or 29, wherein said method is conducted at substantially physiological pH.

31. The method of any of claims 28 to 30, wherein said cells are fixed to an insoluble support matrix before being contacted by said conjugate and said reducing agent.

32. A method of detecting peroxidative injury to living cells comprising:

contacting living cells suspected of having suffered peroxidative injury with a conjugate comprised of a heme protein, a polyamino compound, a luminescer and serum albumin, said luminescer being non-covalently bonded to said serum albumin;

contacting said cells with a reducing agent;

removing any of said conjugate which does not bind to said cells; and

heating said cells.

33. The method of claim 32, wherein said conjugate is soluble in polar solvents.

34. The method of claims 32 or 33, wherein said method is conducted at substantially physiological pH.

35. The method of any of claims 32 to 34, wherein said cells are fixed to an insoluble support matrix before being contacted by said conjugate and said reducing agent.

36. The method of any of claims 32 to 25, wherein said reducing agent is potassium borohydride.

37. The method of any of claims 32 to 36, wherein said luminescer is luminol, a luminol derivative or derivatives, phenol pyruvate, indole-3-pyruvate, tryptophan, N-acetyltryptophan or other tryptophan derivatives, indole propionic acid or indole acetic acid.

38. The method of any of claims 32 to 37, wherein said conjugate is crosslinked with a bifunctional crosslinking agent to form a soluble conjugate.

39. The method of any of claims 32 to 38, wherein said polyamino compound is lysine, spermidine, putresine,

hexene diamine or ethylene diamine.

40. A composition for detection and quantification of peroxidative injury to cells comprising a heme protein, a polyamino compound, a luminescer and serum albumin, said luminescer being non-covalently bonded to said serum albumin and to form a polyamino compound-heme protein-serum albumin-luminescer conjugate.

41. The composition of claim 40, wherein said conjugate is crosslinked by a bifunctional crosslinking agent.

42. The composition of claims 40 or 41, wherein said luminescer is luminol, a luminol derivative or derivatives, phenol pyruvate, indole-3-pyruvate, tryptophan, N-acetyltryptophan or other tryptophan derivatives, indole propionic acid or indole acetic acid.

43. The composition of any of claims 40 to 42, wherein said heme protein is hemoglobin,myoglobin or a peroxidase.

44. The composition of any of claims 40 to 43, wherein said polyamino compound is lysine, spermidine, putresine, hexene diamine or ethylene diamine.

45. A composition for use in a thermochemiluminescent assay comprising a conjugate comprised of a luminescer, a heme protein and serum albumin, said luminescer being non-covalently bonded to said serum albumin.

46. The composition of claim 45, wherein said luminescer is luminol, a luminol derivative or derivatives, phenol pyruvate, indole-3-pyruvate, tryptophan, N-acetyltryptophan or other tryptophan derivatives, indole propionic acid or indole acetic acid.

47. The composition or claims 45 or 46, wherein said heme protein is hemoglobin,myoglobin, or a peroxidase.

48. The composition as claimed in any of claims 45 to 47, comprising a complex in which said heme protein is bonded to said serum albumin.

49. A composition as claimed in claim 45, comprising luminol, a peroxidase and serum albumin, said luminol being non-covalently bonded to said serum albumin.

50. A chemiluminescent assay for the presence of a ligand in a fluid, wherein said ligand is a member of a specific binding pair consisting of said ligand and its antiligand comprising:

admixing a luminescer, a selected antiligand and serum albumin under conditions promoting the non-covalent bonding of said serum albumin to said luminescer to form a soluble antiligand-serum albumin-luminescer conjugate;

contacting said conjugate with a fluid suspected of containing a ligand,wherein said ligand and said antiligand are members of a specific binding pair consisting of said ligand and said antiligand;

removing any unbound ligand; and

triggering a light-generating reaction.

51. The assay of claim 50, wherein said ligand is either a substrate or an enzyme specific for a selected substrate and said antiligand is either an enzyme specific for said substrate or said selected substrate.

52. The assay of claims 50 or 51, wherein said ligand is either an antigen or an antibody specific for a selected antigen and said antiligand is either an antibody specific for said antigen or said selected antigen.

53. The assay of any of claims 50 to 52, wherein said conjugate is additionally comprised of a heme protein and said light-generating reaction is triggered by heat.

54. The assay of any of claims 50 to 52, wherein said light-generating reaction is triggered by contacting said conjugate to which said ligand is bound with a base.

55. The assay of claim 54, wherein said base is sodium hydroxide.

56. The assay of any of claims 50 to 55, wherein said conjugate is immobilized before being contacted with said fluid suspected of containing said ligand.

57. The assay of claim 56, wherein said conjugate is immobilized by crosslinking with a bifunctional crosslinking agent.

58. The assay of claims 56 or 57, wherein said conjugate is immobilized after being contacted with said fluid suspected of containing said ligand.

59. The assay of any of claims 50 to 55, wherein said ligand is immobilized.

60. The assay of claim 59, wherein said ligand is immobilized by binding to an insoluble support matrix.

61. A method of detecting a substrate, wherein the breakdown of said substrate is catalyzed by a dehydrogenase comprising:

admixing a luminescer, a dehydrogenase specific for a selected substrate and serum albumin under conditions promoting the bonding of said dehydrogenase to said serum albumin and the non-covalent bonding of said luminescer to said serum albumin to form a dehydrogenase-serum albumin-luminescer conjugate;

contacting said conjugate with a solution containing a bifunctional crosslinking agent and an oxidized nucleotide cofactor to form a gel;

contacting said gel with a sample suspected of containing said selected substrate; and

triggering a light-generating reaction.

62. The method of claim 61, wherein said conjugate is additionally comprsed of a heme protein and said light-generating reaction is triggered by heat.

63. The method of claim 61, wherein said light-generating reaction is triggered by contacting said gel with a base.

64. A composition for use in a chemiluminescent assay for a specific analyte wherein said analyte is a member of a specific binding pair comprising a conjugate comprised of a heme protein, a luminescer and serum albumin, said luminescer being non-covalently bonded to said serum albumin, said conjugate being crosslinked in a bifunctional crosslinking agent.

65. The composition of claim 64, wherein said luminescer is luminol, a luminol derivative or derivatives, phenol

pyruvate, indole-3-pyruvate, tryptophan, N-acetyltryptophan or other tryptophan derivatives, indole propionic acid or indole acetic acid.

66. An assay for detection of an antigen in a fluid, wherein said antigen is a member of a specific binding pair consisting of an antigen and an antibody comprising:

binding a first antibody to an insoluble support matrix, said first antibody being specific for a first determinant on an antigen which is desired to be detected;

contacting said first antibody with a fluid suspected of containing an antigen which is desired to be detected;

contacting antigen bound to said first antibody with a conjugate comprising an antibody specific for a second determinant on said antigen, serum albumin and a luminescer, said luminescer being non-covalently bonded to said serum albumin, under conditions promoting the binding of said second antibody of said conjugate to said antigen; and

triggering a light-generating reaction.

67. The assay of claim 66, wherein said insoluble support matrix is comprised of an inert polymer.

68. The assay of claim 67, wherein said inert polymer is a crosslinked protein.

69. The assay of any of claims 66 to 68, wherein unbound conjugate is removed before said light-generating reaction is triggered.

70. The assay of any of claims 66 to 69, wherein said luminescer is luminol, a luminol derivative or derivatives, phenol pyruvate, indole-3-pyruvate, tryptophan, N-acetyltryptophan or other tryptophan derivatives, indole propionic acid or indole acetic acid.

71. The assay of any of claims 66 to 70, wherein said first antibody is bound to protein A, said protein A being bound to an insoluble support matrix.

72. An assay as claimed in claim 71, in which the assay is a chemiluminescent assay comprising contacting antigen bound to said first antibody comprising protein A, a

second antibody specific for a second determinant on said antigen, serum albumin and luminol, said luminol being non-covalently bonded to said serum albumin, under conditions prompting the binding of said second antibody of said conjugate to said antigen.

73. The assay of claims 71 or 72, wherein said insoluble support matrix is comprised of a polysaccharide.

74. The assay of any of claims 66 to 73, wherein said light-generating reaction is triggered with a base.

75. The assay of any of claims 66 to 73, wherein said conjugate is additionally comprised of a heme protein and said light-generating reaction is triggered by heat.

76. A reagent for use in a chemiluminescent assay for detection of an antigen in a fluid, wherein said antigen is a member of a specific binding pair consisting of an antigen and an antibody specific for that antigen comprising a conjugate of serum albumin, protein A and a luminescer, said luminescer being non-covalently bonded to said serum albumin.

77. The reagent of claim 76, wherein said luminescer is luminol, a luminol derivative or derivatives, phenol pyruvate, indole-3-pyruvate, tryptophan, N-acetyltryptophan or other tryptophan derivatives, indole propionic acid or indole acetic acid.

78. The reagent of claims 76 or 77, additionally comprising a heme protein.

79. A chemiluminescent assay for detection of a selected antibody in a fluid wherein said antibody is a member of a specific binding pair consisting of an antigen and an antibody specific for that antigen comprising:

binding an antigen to an insoluble support matrix, said antigen having a determinant to which a selected antibody will bind;

contacting said bound antigen with a fluid suspected of containing said selected antibody under conditions promoting the binding of said antibody to said antigen;

contacting said bound antibody with a conjugate comprising protein A, serum albumin and a luminescer, said luminescer being non-covalently bonded to said serum albumin, under conditions promoting the binding of the protein A of said conjugate to said bound antibody; and

triggering a light-generating reaction.

80. The assay of claim 79, wherein said luminescer is luminol, a luminol derivative or derivatives, phenol pyruvate, indole-3-pyruvate, tryptophan, N-acetyltryptophan or other tryptophan derivatives, indole propionic acid or indole acetic acid.

81. The assay of claims 79 or 80, wherein said antigen is bound to said insoluble support matrix by crosslinking with a bifunctional crosslinking agent to form a gel.

82. The assay of any of claims 79 to 81, wherein said bifunctional crosslinking agent is glutaraldehyde, a carbodiimide, succinyl anhydride or N-succinimidyl-3-[2-pyridyldithio]-propionate.

83. The assay of any of claims 79 to 82, wherein said light-generating reaction is triggered with a base.

84. The assay of any of claims 79 to 82, wherein said luminescent reagent additionally comprises a heme protein and said light-generating reaction is triggered by heat.

85. The assay of claim 84, wherein said heme protein is hemoglobin, myoglobin or a peroxidase.

86. The assay of any of claims 79 to 85, wherein said fluid suspected of containing a specific antigen is blood serum.

87. The assay of any of claims 79 to 86, wherein said unbound conjugate is removed before said light-generating reaction is triggered.

88. A chemiluminescent assay for detection of a selected antibody in a fluid,wherein said antibody is a member of a specific binding pair comprising said antibody and an antigen specifically bound by that said antibody comprising:

binding an antigen to an insoluble support matrix,

said antigen having a determinant to which a selected antibody will bind;

contacting said bound antigen with a fluid suspected of containing said selected antibody under conditions promoting the binding of said antibody to said bound antigen;

contacting said bound antibody with a conjugate comprising protein A, serum albumin and luminol, said luminol being non-covalently bonded to said serum albumin, under conditions promoting the binding of the protein A of said conjugate to said bound antibody; and

contacting bound conjugate with base.

89. The chemiluminescent assay of claim 88, wherein said fluid suspected of containing said antibody is blood serum.

90. A chemiluminescent assay for detection of a selected antibody in a fluid, wherein said antibody is a member of a specific binding pair comprising said antibody and an antigen specifically bound by said antibody comprising:

binding an antigen to an insoluble support matrix, said antigen having a determinant to which a selected antibody will bind;

contacting said bound antigen with a fluid suspected of containing said selected antibody under conditions promoting the binding of said antibody to said bound antigen;

contacting said bound antibody with a conjugate comprising a heme protein, protein A, serum albumin and luminol, said luminol being non-covalently bonded to said serum albumin, under conditions promoting the binding of the protein A of said conjugate to said bound antibody; and

heating said bound conjugate.

91. The chemiluminescent assay of claim 90, wherein said fluid suspected of containing said antibody is a body fluid.

92. An assay for the detection of a selected type of cell comprising:

immobilizing a sample suspected of containing a selected type of cell;

contacting said immobilized cells with a conjugate comprising a lectin, serum albumin, and a luminescer in a bifunctional crosslinking agent, said lectin being selected for its ability to preferentially bind to said selected type of cell, and said luminescer being non-covalently bonded to said serum albumin, under conditions promoting the binding of the lectin of said conjugate to said selected type of cell; and

triggering a light-generating reaction.

93. The assay of claim 92, wherein said light-generating reaction is triggered by contacting said bound conjugate with a base.

94. The assay of claim 92, wherein said conjugate additionally comprises a heme protein and said light-generating reaction is triggered by heat.

95. The assay of any of claims 92 to 94, wherein said fluid suspected of containing a selected type of cell is a body fluid.

96. The assay of any of claims 92 to 95, wherein said luminescer is luminol, a luminol derivative or derivatives, phenol pyruvate, indole-3-pyruvate, tryptophan, N-acetyltryptophan or other tryptophan derivatives, indole propionic acid or indole acetic acid.

97. The assay of any of claims 92 to 96, wherein said bifunctional crosslinking agent is glutaraldehyde, a carbodiimide, succinyl anhydride or N-succinimidyl-3-[2-pyridyldithio]-propionate.

98. The assay of any of claims 92 to 97, wherein said lectin is concanavalin A, wheat germ agglutinin, lentil lectin, soybean lectin, or Helix pomatia lectin.

99. The assay of any of claims 92 to 98, wherein unbound conjugate is removed before said light-generating reaction is triggered.

100. A composition for detecting a selected cell type comprising a conjugate of serum albumin, a lectin and a luminescer, said luminescer being non-covalently bonded to said serum albumin.

101. The composition of claim 100, wherein said luminescer is luminol, a luminol derivative or derivatives, phenol pyruvate, indole-3-pyruvate, tryptophan, N-acetyltryptophan or other tryptophan derivatives, indole propionic acid or indole acetic acid.

102. The composition of any of claims 100 or 101, wherein said lectin is concanavalin A, wheat germ agglutinin, lentil lectin, soybean lectin or Helix pomatia lectin.

103. The composition of any of claims 100 to 102, additionally comprising a heme protein.

104. A device for detection of a specific cell type comprising a lectin-serum albumin-luminescer conjugate, said luminescer being non-covalently bonded to said serum albumin, said conjugate being crosslinked with a bifunctional crosslinking agent.

105. The device of claim 104, wherein said luminescer is luminol, a luminol derivative or derivatives, phenol pyruvate, indole-3-pyruvate, tryptophan, N-acetyltryptophan or other tryptophan derivatives, indole propionic acid or indole acetic acid.

106. The device of any of claims 104 or 105, wherein said lectin is concanavalin A, wheat germ agglutinin, lentil lectin, soybean lectin or Heliz pomatia lectin.

107. The device of any of claims 104 to 106, wherein said bifunctional crosslinking agent is glutaraldehyde, a carbodiimide, succinyl anhydride or N-succinimidyl-3-[2-pyridyldithio]-propionate.

108. The device of any of claims 104 to 107, wherein said conjugate additionally comprises a heme protein.

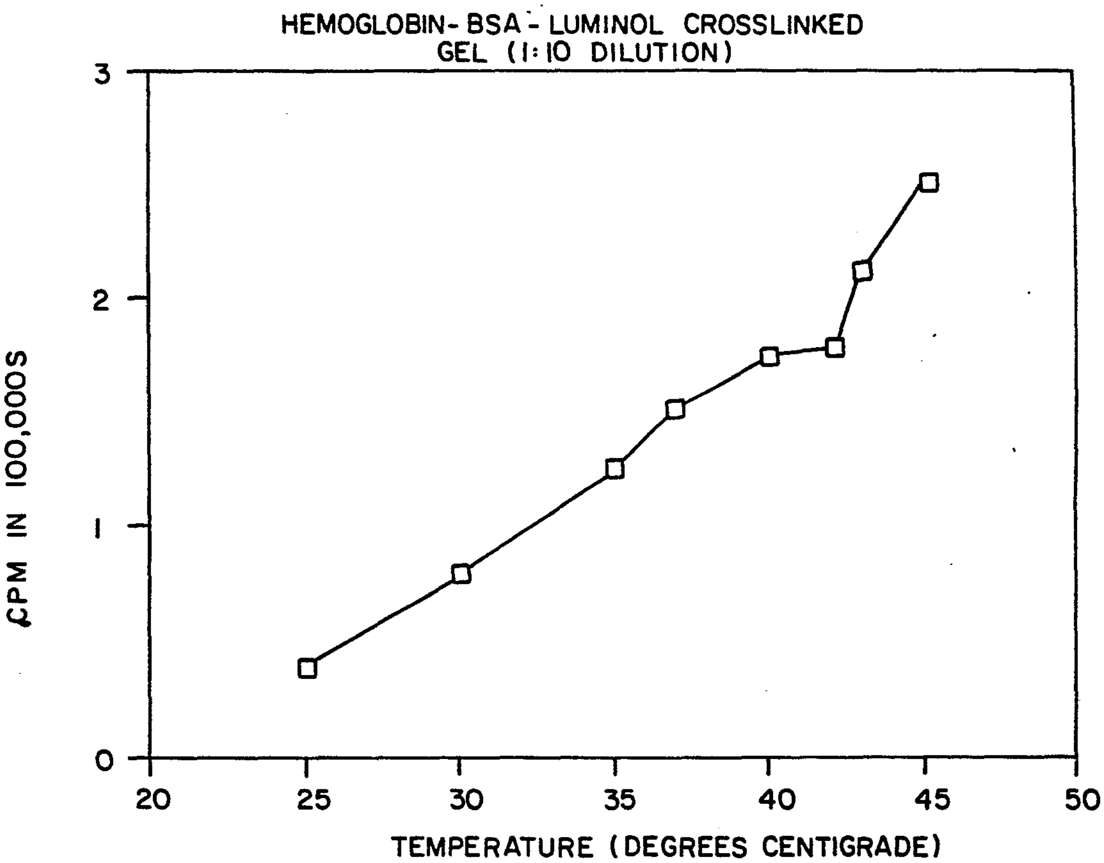
HEMOGLOBIN-BSA-LUMINOL CROSSLINKED
GEL (1:10 DILUTION)
CPM IN 100,000S
TEMPERATURE (DEGREES CENTIGRADE)
0
1
2
3
20
25
30
35
40
45
50

FIG.1

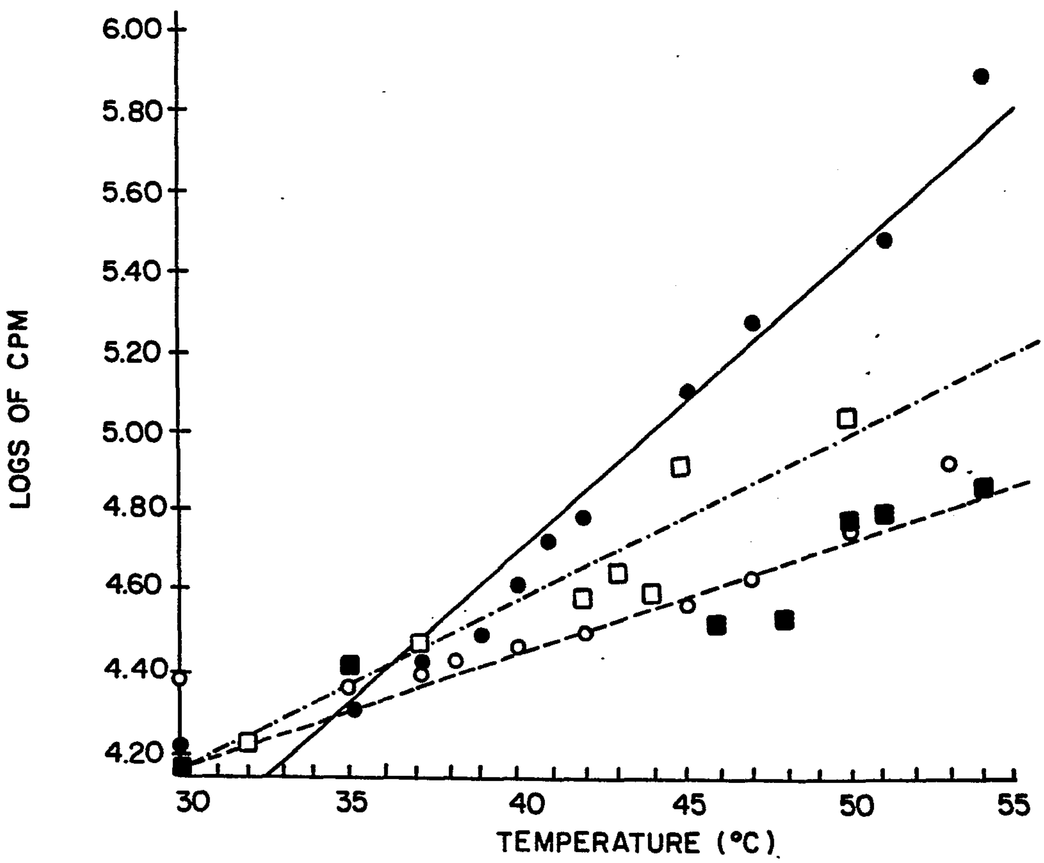
LOGS OF CPM
TEMPERATURE (°C)
6.00
5.80
5.60
5.40
5.20
5.00
4.80
4.60
4.40
4.20
30
35
40
45
50
55

FIG.2

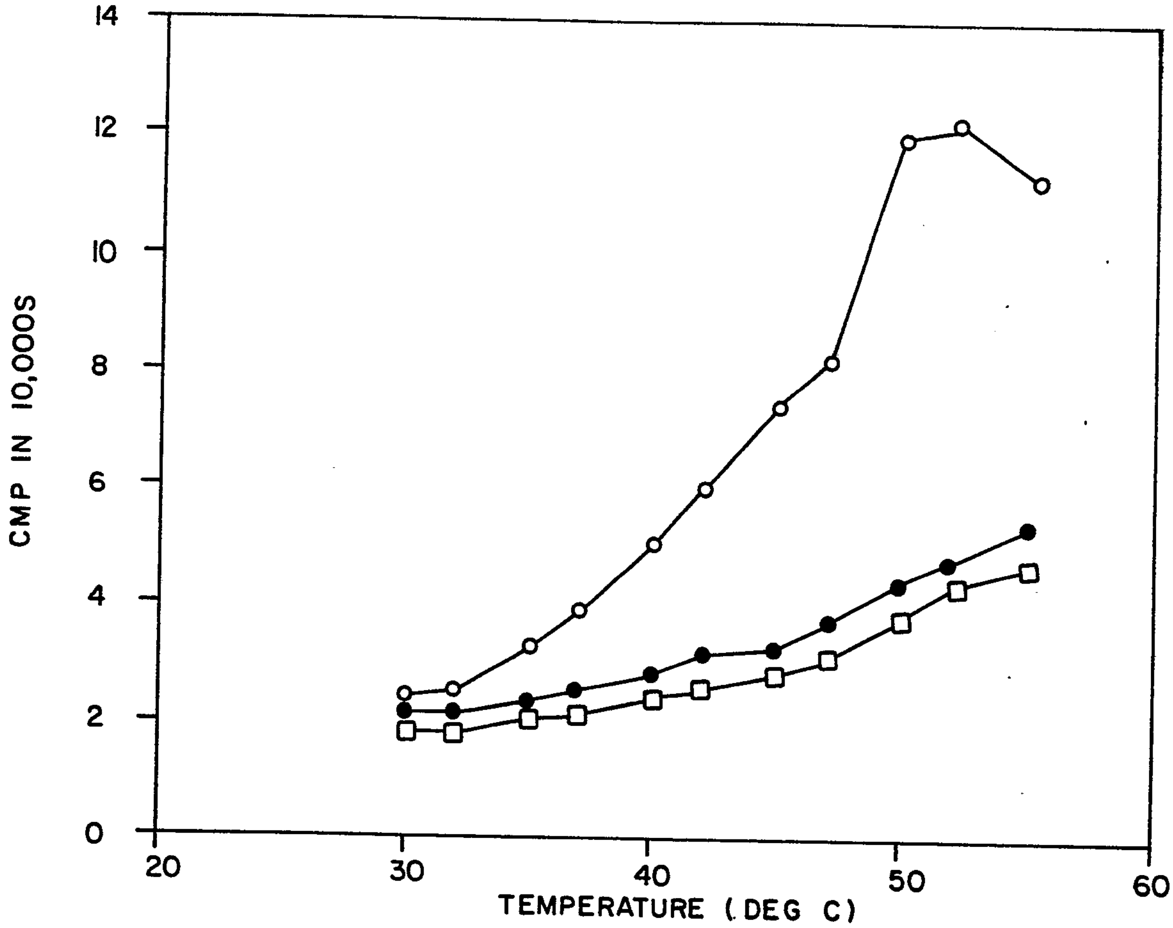
14
12
10
8
6
4
2
0
CMP IN 10,000S
20
30
40
50
60
TEMPERATURE (DEG C)

FIG.3

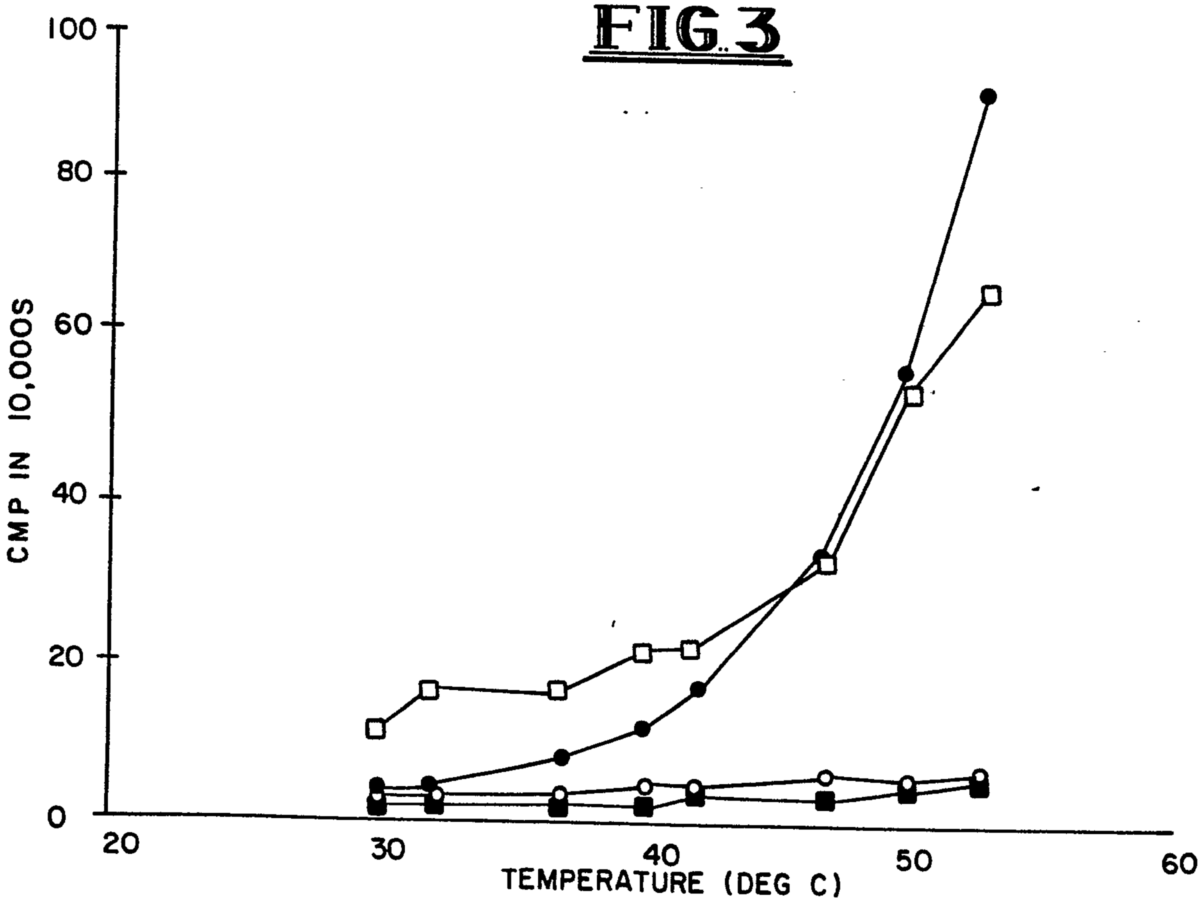
100
80
60
40
20
0
CMP IN 10,000S
20
30
40
50
60
TEMPERATURE (DEG C)

FIG.4

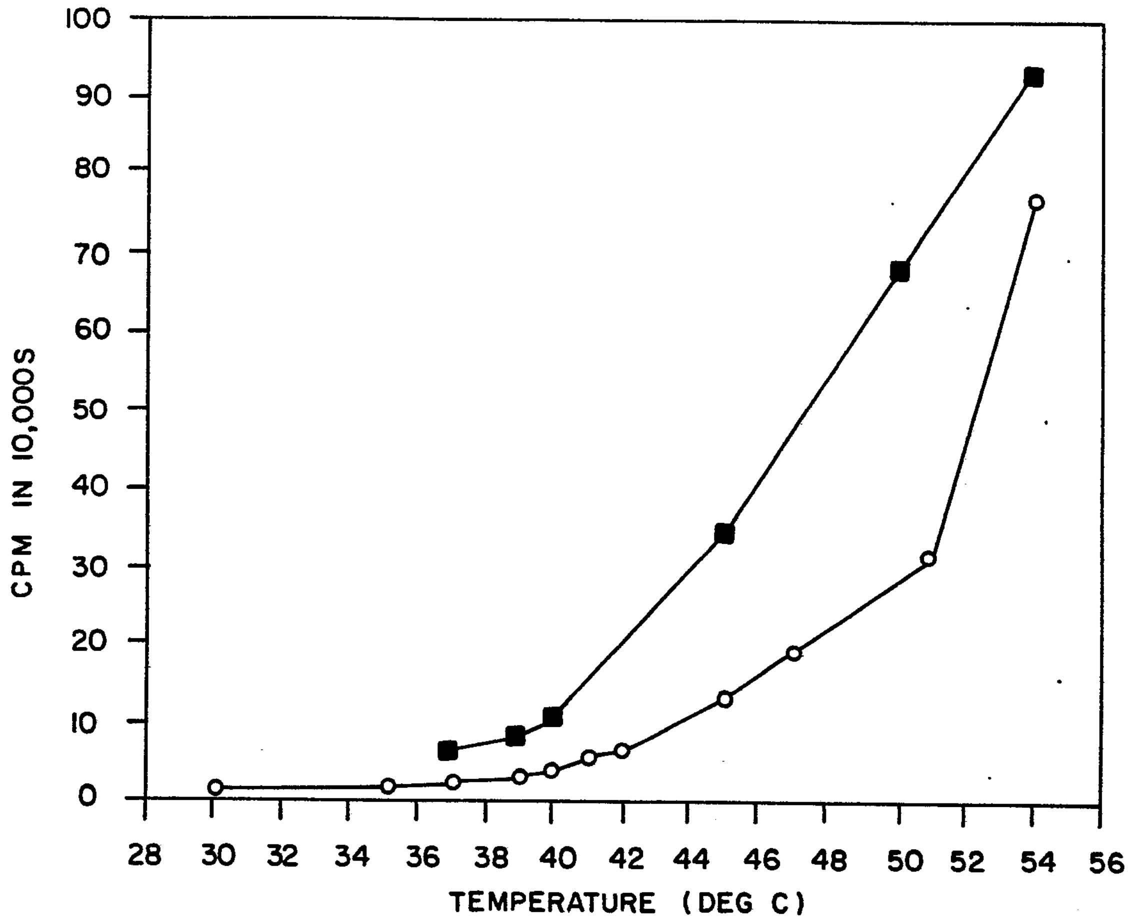
100
90
80
70
60
50
40
30
20
10
0
CPM IN 10,000S
28 30 32 34 36 38 40 42 44 46 48 50 52 54 56
TEMPERATURE (DEG C)

FIG 5

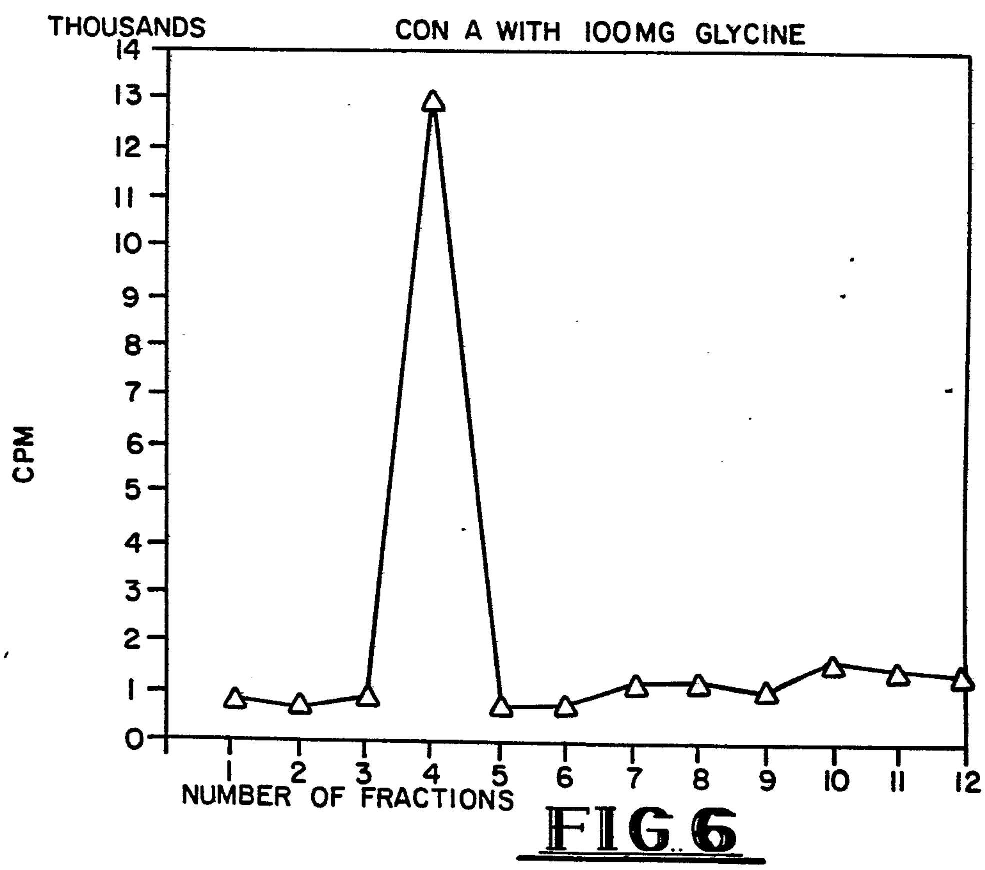
THOUSANDS
CON A WITH 100MG GLYCINE
14
13
12
11
10
9
8
7
6
5
4
3
2
1
0
CPM
1 2 3 4 5 6 7 8 9 10 11 12
NUMBER OF FRACTIONS

FIG 6

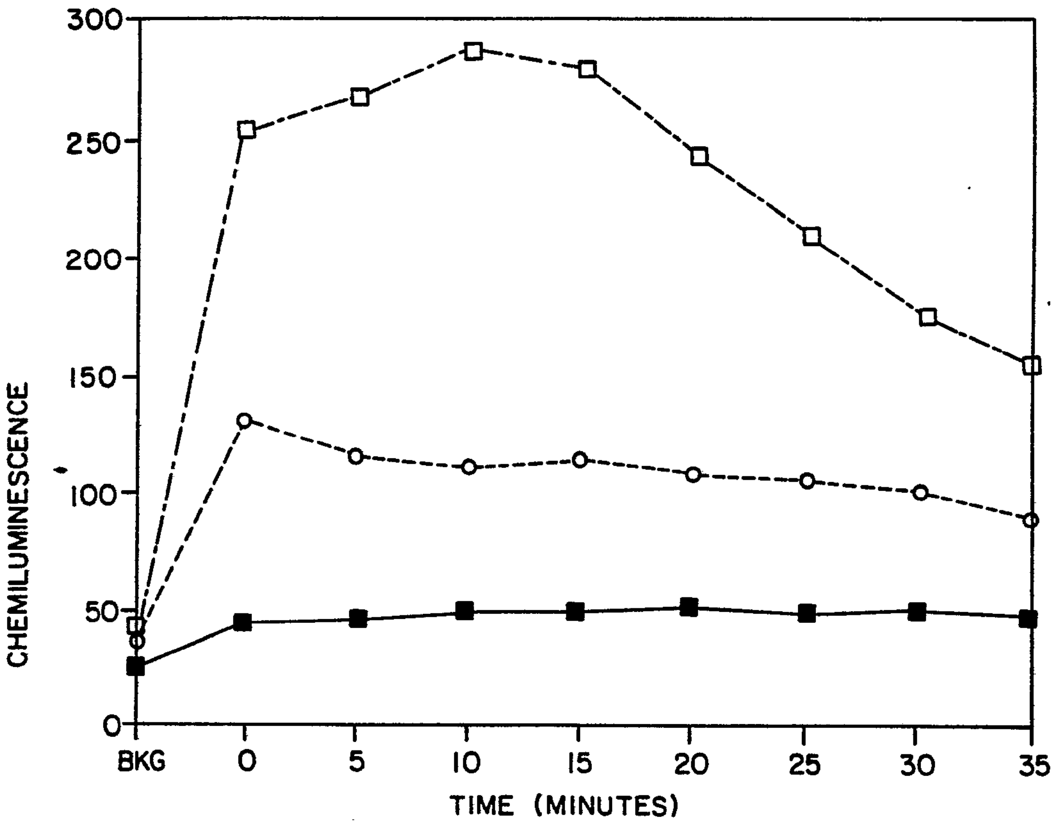
300
250
200
150
100
50
0
CHEMILUMINESCENCE
BKG
0
5
10
15
20
25
30
35
TIME (MINUTES)

FIG 7